# FASCICULE DE BREVET EUROPEEN

(11) **EP 0 944 618 B1**
(45) Date de publication et mention de la délivrance du brevet: **24.09.2003**
(21) Numéro de dépôt: 97945928.6
(22) Date de dépôt: 14.11.1997
(51) Int. Cl.: C07D 401/12, A61K 31/47

(54) **NOUVEAUX COMPOSES DE N-BENZENESULFONYL-L-PROLINE, PROCEDE DE PREPARATION ET UTILISATION EN THERAPEUTIQUE**
N-BENZOLSULFONYL-L-PROLIN DERIVATE, VERFAHREN ZU IHRER HERSTELLUNG UND IHRE THERAPEUTISCHE VERWENDUNG
NOVEL N-BENZENESULPHONYL-L-PROLINE DERIVATIVES, METHOD FOR PREPARING AND THERAPEUTIC USE

(30) Priorité: 04.12.1996 FR 9614891
(43) Date de publication de la demande: 29.09.1999
(73) Titulaire: FOURNIER INDUSTRIE ET SANTE, F-75008 Paris (FR)
(72) Inventeur: DODEY, Pierre, F-21121 Fontaine-lès-Dijon (FR); BONDOUX, Michel, F-21121 Fontaine-lès-Dijon (FR); OU, Khan, F-21121 Hauteville-lès-Dijon (FR); BARTH, Martine, F-21000 Dijon (FR); HOUZIAUX, Patrick, F-78580 Bazemont (FR)
(74) Mandataire: Hubert, Philippe
(86) Numéro de dépôt international: FR9702049
(87) Numéro de publication internationale: WO98024783

(56) Documents cités:
- EP-A- 0 622 361

## Description

### Domaine de l'invention

La présente invention concerne de nouveaux composés dérivés de la N-(benzènesulfonyl)-(L)-proline, leur procédé de préparation et leur utilisation en thérapeutique.

Ces nouveaux composés présentent une action antagoniste vis-à-vis de la bradykinine et sont utiles en thérapeutique, particulièrement pour le traitement de la douleur et de l'inflammation, et notamment dans le traitement de l'asthme, du choc traumatique cérébral et des rhinites allergiques.

### Art antérieur

On sait que l'une des possibilités de traitement de certaines pathologies à caractère douloureux et/ou inflammatoire (telles que l'asthme, la rhinite, le choc septique, la douleur dentaire, etc.) est d'inhiber l'action de certaines hormones telles que la bradykinine ou la kallidine. En effet ces hormones peptidiques sont impliquées dans un grand nombre de processus physiologiques dont certains sont liés de façon étroite à ces pathologies.

Bien qu'actuellement aucun produit possédant ce mode d'action ne soit encore commercialisé, de nombreuses études ont été entreprises pour comprendre le mode d'action des kinines et en particulier la bradykinine et ses homologues, puis pour créer des composés susceptibles d'être antagonistes des récepteurs de la bradykinine. Parmi les nombreuses publications relatant ces travaux, on peut citer Pharmacological Reviews Vol. 44 n° 1, pages 1-80 (1992) et Biopolymers (Peptide Science) vol. 37 pages 143-155 (1995).

La bradykinine est une hormone peptidique constituée de 9 aminoacides (Arg-Pro-Pro-Gly-Phe-Ser-Pro-Phe-Arg) et la kallidine est une hormone peptidique (Lys-Arg-Pro-Pro-Gly-Phe-Ser-Pro-Phe-Arg) qui comporte un aminoacide supplémentaire (Lys) par rapport à la bradykinine. On sait que des études antérieures ont permis d'obtenir des peptides qui interagissent avec les récepteurs de la bradykinine : certains comme le Bradycor (CP.0127 de la société CORTECH), l'Icatibant (HOE 140 de la société HOECHST) ["Bradycor" et "Icatibant" sont des dénominations communes internationales (DCI)] ou encore le NPC 17761 (de la société SCIOS-NOVA) présentent une action inhibitrice de la fixation de la bradykinine sur son récepteur B₂. Plus récemment, des composés non peptidiques ont été proposés comme antagonistes vis-à-vis de la fixation de la bradykinine sur son récepteur B₂, notamment dans EP-A-0596406 et EP-A-0622361. On sait en outre que certains composés de structure apparentée à celles des composés visés dans les deux demandes de brevet précitées ont déjà été décrits, notamment dans les publications DE-A-3617183 et dans EP-A-0261539, eu égard à leurs éventuelles propriétés antithrombotiques.

### But de l'invention

Il existe un besoin d'atténuer ou de supprimer chez les mammifères et surtout chez l'homme les douleurs et les inflammations.

Pour satisfaire ce besoin, on a recherché une nouvelle solution technique qui soit efficace dans le traitement des algies quelle que soit leur origine, notamment dans le traitement (i) des algies liées à des phénomènes inflammatoires, et (ii) des inflammations.

Selon l'invention, on se propose de fournir une nouvelle solution technique, qui met en oeuvre une fixation compétitive au niveau du récepteur B₂ de la bradykinine entre (i) la bradykinine et les hormones apparentées ou analogues, et (ii) une substance antagoniste, et qui fait appel à des composés de type benzènesulfonamide, structurellement différents des produits connus précités, et capables de limiter ou d'inhiber substantiellement la fixation de la bradykinine et des hormones analogues sur ledit récepteur B₂ de la bradykinine.

Suivant cette solution technique, les nouveaux composés se fixent de façon compétitive sur le récepteur B₂ de la bradykinine sans provoquer les effets de la bradykinine sur ce récepteur (la substance est dite antagoniste). Il s'en suit l'apparition d'un état analogue à celui observé en l'absence de bradykinine, à savoir une diminution de la douleur et des réactions inflammatoires.

Conformément à cette nouvelle solution technique on se propose de fournir, selon un premier aspect de l'invention, des composés dérivés de N-(benzènesulfonyl)-(L)-proline en tant que produits industriels nouveaux ; selon un second aspect de l'invention, un procédé de préparation de ces composés ; et selon un troisième aspect de l'invention, une utilisation de ces composés notamment en thérapeutique en tant qu'agents antalgiques et/ou anti-inflammatoires.

### Objet de l'invention

Selon la nouvelle solution technique de l'invention, on préconise en tant que produit industriel nouveau, un composé de N-(benzènesulfonyl)-L-proline qui est caractérisé en ce qu'il est choisi parmi l'ensemble constitué par :

### (i) les composés de formule I :

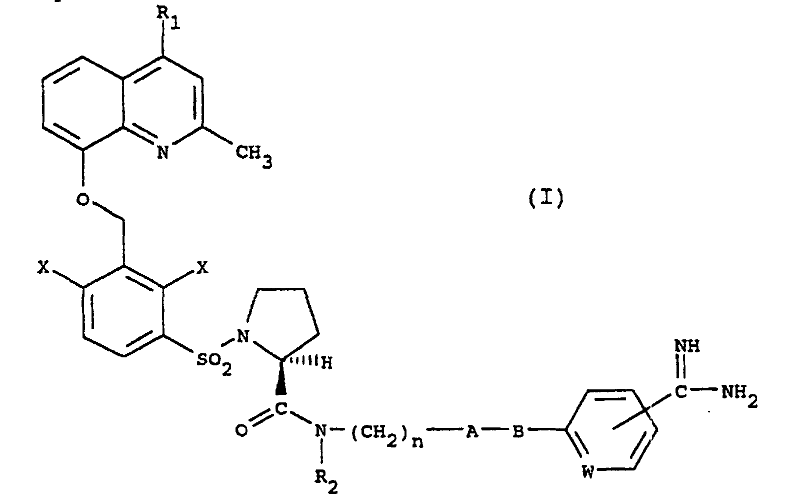

dans laquelle :
- X: représente un atome d'halogène ou un groupe méthyle,
- A: représente un groupe -N(R₃)-CO- ou -CO-N(R₃)-,
- B: représente une liaison simple, -CH₂- ou -CH₂-O-,
- R₁: représente un atome d'hydrogène, un groupe alkyle en C₁-C₃ à chaîne hydrocarbonée linéaire ou ramifiée, ou un groupe trifluorométhyle,
- R₂ et R₃: représentent chacun indépendamment un atome d'hydrogène ou un groupe alkyle en C₁-C₃ à chaîne hydrocarbonée linéaire ou ramifiée,
- W: représente CH (le noyau aromatique est alors un groupe phényle) ou N (le noyau aromatique est alors un groupe pyridinyle),
- n: représente 2, 3, 4 ou 5 ; et,

### (ii) leurs sels d'addition.

Selon l'invention, on préconise aussi un procédé de préparation des composés de formule I et de leurs sels d'addition.

On préconise également l'utilisation d'une substance antagoniste d'un récepteur de la bradykinine et des hormones analogues à la bradykinine, ladite utilisation étant caractérisée en ce que l'on fait appel à une substance antagoniste du récepteur B₂ de la bradykinine et choisie parmi les composés de formule I et leurs sels d'addition non-toxiques, pour l'obtention d'un médicament destiné à une utilisation en thérapeutique vis-à-vis des pathologies impliquant la bradykinine ou ses analogues, en particulier vis-à-vis des algies, et notamment dans le traitement ou la prévention de pathologies liées à des états inflammatoires ou douloureux.

### Description détaillée de l'invention

Dans la formule générale I des composés de l'invention, on entend par atome d'halogène un atome de fluor, de chlore, de brome ou d'iode, l'halogène préféré étant l'atome de chlore.

Par groupe alkyle en C₁-C₃ à chaîne hydrocarbonée linéaire ou ramifiée, on entend ici le groupe méthyle, éthyle, propyle ou 1-méthyléthyle.

Dans le composé de formule I, l'hétérocycle azoté de type pyrrolidine comprend 1 atome de carbone asymétrique. Selon l'invention, ce carbone est de configuration S, ce qui correspond à la configuration de la L-proline.

Comme indiqué dans la formule générale des composés selon l'invention, la structure comprend un groupe amidine -C(=NH)NH₂ sur un noyau aromatique ; ce groupe amidine peut occuper toute position substituable sur son noyau aromatique, en particulier il peut se situer en position 2, 3, ou 4 lorsque ledit noyau est phényle ou en position 3, 4, 5, ou 6 lorsque ledit noyau est pyridinyle.

Par "sels d'addition", on entend les sels d'addition d'acide obtenus par réaction d'un composé de formule I avec un acide minéral ou un acide organique. Les acides minéraux préférés pour salifier un composé basique de formule I sont les acides chlorhydrique, bromhydrique, phosphorique et sulfurique. Les acides organiques préférés pour salifier un composé basique de formule I sont les acides méthanesulfonique, benzènesulfonique, maléique, fumarique, oxalique, citrique, lactique et trifluoroacétique.

Le procédé général de préparation des composés de formule I, que l'on préconise selon l'invention comprend les étapes consistant à :
(1) faire réagir un acide de formule II : 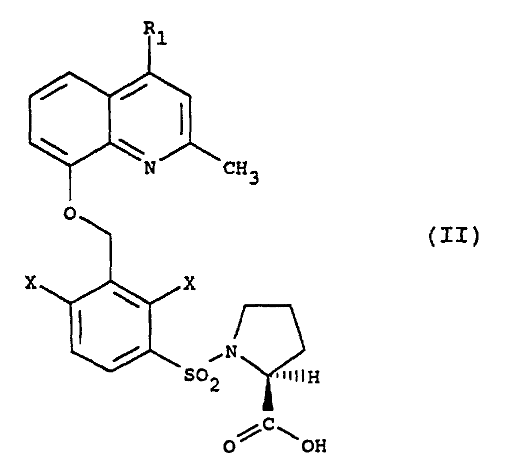 dans laquelle :
   - R₁: représente un atome d'hydrogène, un groupe alkyle en C₁-C₃ à chaîne hydrocarbonée linéaire ou ramifiée ou un groupe trifluorométhyle,
   - X: représente un halogène ou un groupe méthyle,
   avec une amine de formule III : 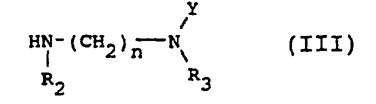
   dans laquelle
   - R₂ et R₃: représentent chacun indépendamment un atome d'hydrogène ou un groupe alkyle en C₁-C₃,
   - n: représente 2, 3, 4 ou 5, et
   - Y: représente un groupe amino-protecteur, par exemple le groupe Boc (1,1-diméthyléthoxycarbonyle),
   dans un solvant, tel que par exemple le dichlorométhane ou le diméthylformamide, en présence d'au moins un activateur couramment utilisé pour créer des liaisons de type peptidique tel que par exemple le chlorhydrate de 1-(3-diméthylaminopropyl)-3-éthyl-carbodiimide (EDCI) et le 1-hydroxy-7-azabenzotriazole (HOAT), à une température proche de la température ambiante (i.e. une température comprise entre environ 0 et environ 40°C, et mieux une température comprise entre 10 et 35°C), pendant 2 à 50 heures, pour obtenir un composé de formule IV : 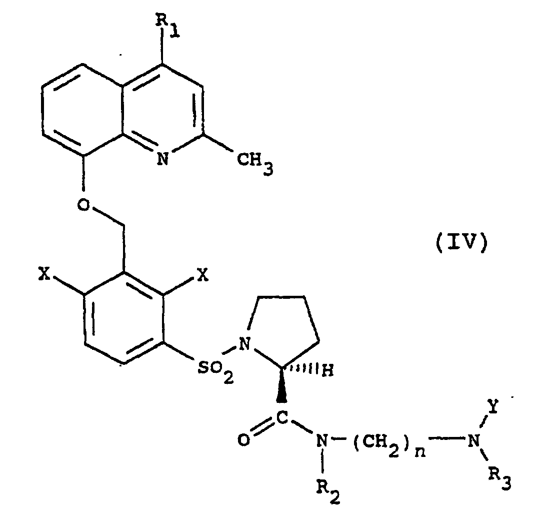 dans laquelle R₁, R₂, R₃, X, Y et n conservent la même signification que ci-dessus ;
(2) déprotéger le composé de formule IV, ainsi obtenu, de façon à remplacer le groupe protecteur Y par un atome d'hydrogène, par exemple, si Y est le groupe Boc, en faisant réagir le composé de formule IV avec de l'acide trifluoroacétique, dans un solvant tel que le dichlorométhane ou l'acétate d'éthyle, à température ambiante (15-25 °C), pendant 5 à 30 heures, pour obtenir le composé de formule V : 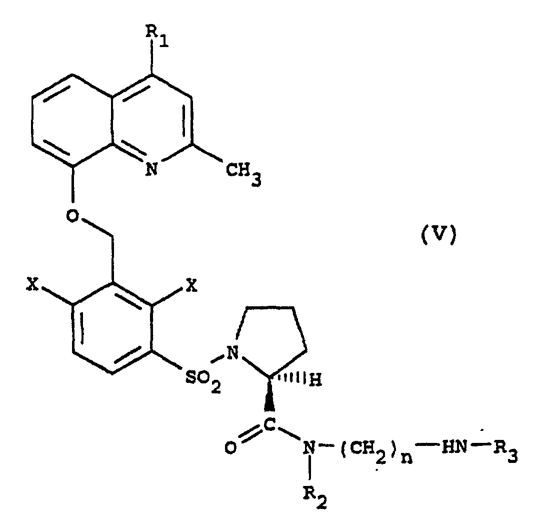 dans laquelle R₁, R₂, R₃, X et n conservent la même signification que ci-dessus ; (en variante de ce procédé, le remplacement du groupe Boc peut être obtenu par action d'une solution d'acide chlorhydrique dans l'eau, en laissant réagir le composé de formule IV pendant 1 à 10 heures, à une température comprise entre 30 et 60°C ; on obtient dans ce cas le composé de formule V sous forme de dichlorhydrate) ;
(3) faire réagir le composé de formule V, ainsi obtenu, avec un acide de formule VI : 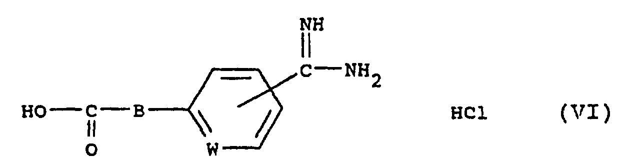 dans laquelle
   - B: représente une liaison simple, un groupe -CH₂- ou -CH₂-O-, et
   - W: représente -CH- ou N,
   dans des conditions analogues à celles préconisées pour l'étape (1) ci-dessus, et, si nécessaire en présence d'une base telle que la N-méthylmorpholine si l'amine de formule V est mise en réaction sous sa forme salifiée, pour obtenir le composé de formule I : 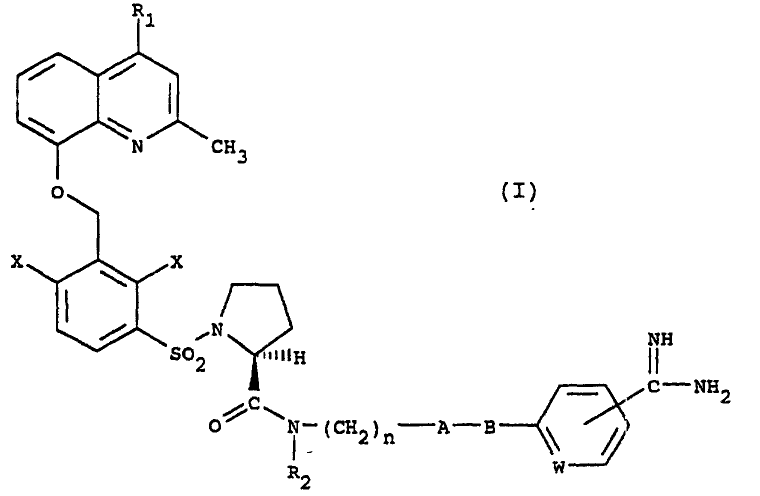 dans laquelle
   - A: représente -N(R₃)-CO-,
   - B: représente une liaison simple, -CH₂- ou -CH₂-O-,
   - R₁: représente un atome d'hydrogène, un groupe alkyle en C₁-C₃ ou un groupe trifluorométhyle,
   - R₂ et R₃: représentent chacun indépendamment H ou un groupe alkyle en C₁-C₃,
   - X: représente un halogène ou un groupe méthyle,
   - W: représente CH ou N, et
   - n: représente 2, 3, 4 ou 5.

Selon une première variante A, on peut obtenir le composé de formule I (dans laquelle A représente -N(R₃)-CO-) par réaction d'un composé de formule V, obtenu selon l'étape (2) ci-dessus, avec un chlorure d'acide de formule VII : 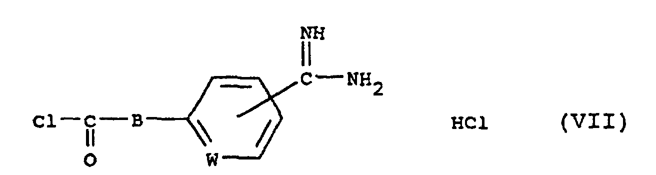 dans laquelle
- B: représente une liaison simple, -CH₂- ou -CH₂-O-,
- W: représente CH ou N,
dans un solvant, tel que par exemple le dichlorométhane, et en présence d'une base aprotique, telle que par exemple la N-méthylmolpholine, à température proche de la température ambiante et pendant 2 à 30 heures.

Selon une seconde variante dénommée B,
(a) on fait réagir le composé de formule V, obtenu à l'étape (2) ci-dessus, avec un composé de formule VIII : 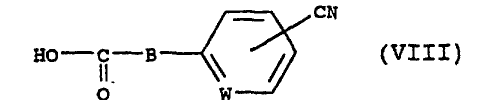 dans laquelle
   - B: représente une liaison simple, -CH₂- ou -CH₂O-, et
   - W: représente CH ou N,
   dans des conditions opératoires analogues à celles de l'étape (3) décrite ci-dessus, pour obtenir un composé de formule IX : 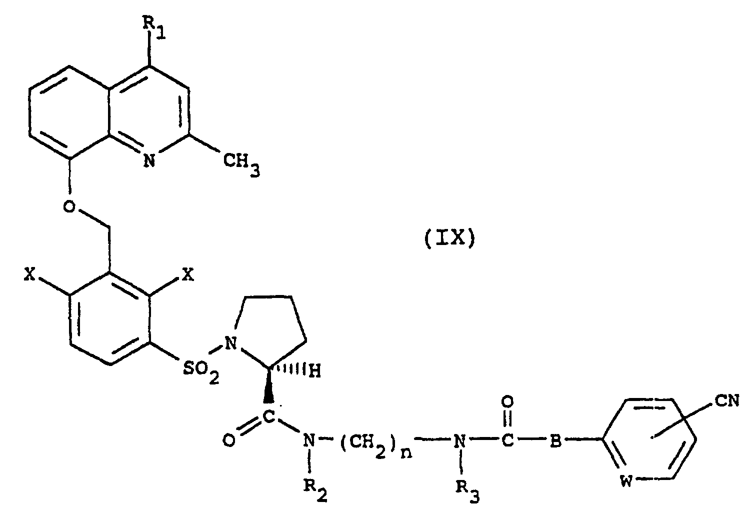 dans laquelle R₁, R₂, R₃, X, W, n et B conservent la même signification que dans les produits de départ ;
(b) on fait réagir le composé de formule IX, ainsi obtenu, avec de l'hydroxylamine (libérée à partir de son chlorhydrate dans le milieu réactionnel par action d'une base telle que la triéthylamine), dans un solvant, comme par exemple le diméthylsulfoxide (DMSO), à température ambiante et pendant 2 à 12 heures, pour obtenir le composé de formule X : 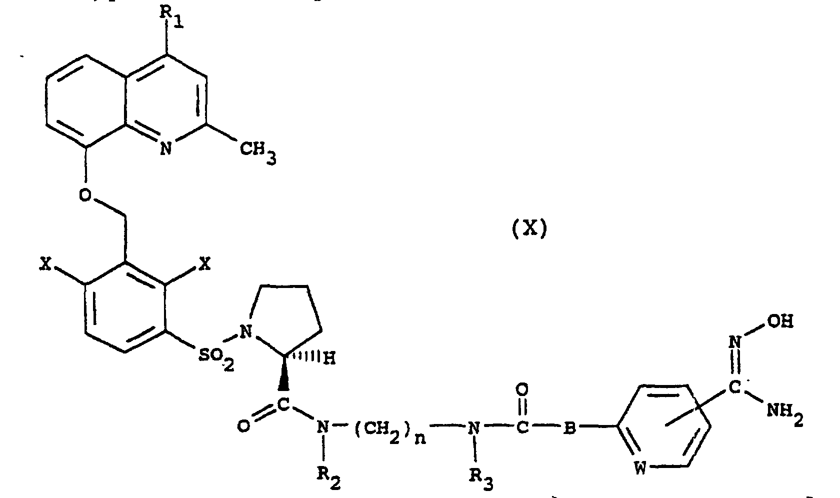 dans laquelle R₁, R₂, R₃, X, W, n et B conservent la même signification que précédemment ;
(c) on fait réagir le composé de formule X, ainsi obtenu, avec un excès d'anhydride acétique, dans un solvant, tel que par exemple le dichlorométhane, à température ambiante pendant 1 à 15 heures, pour obtenir le composé de formule XI : 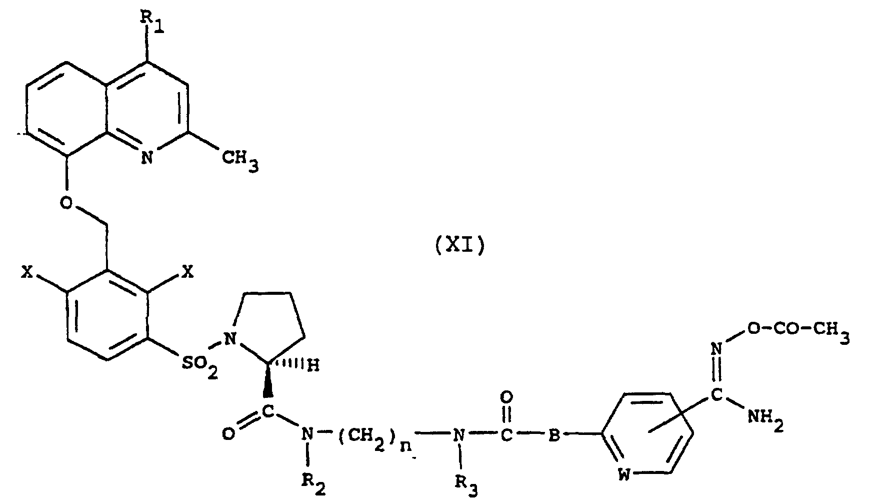 dans laquelle R₁, R₂, R₃, X, W, n et B conservent la même signification que ci-dessus ; et,
(d) on effectue une hydrogénation catalytique du composé de formule XI, ainsi obtenu, dans un solvant, comme par exemple le méthanol, en présence d'un catalyseur d'hydrogénation tel que le catalyseur de Lindlar, à température ambiante, sous une pression d'hydrogène comprise entre 10⁵ et 5.10⁵ Pa, pour obtenir le composé de formule I, dans laquelle R₁, R₂, R₃, X, W, n et B conservent la même signification que précédemment, A représente le groupe -N(R₃)-CO-, et le groupe amidine conserve la position initiale du groupe cyano de l'acide de départ.

Selon une troisième variante dénommée C du procédé de préparation d'un composé selon l'invention, on préconise de
(α) faire réagir un acide de formule II : 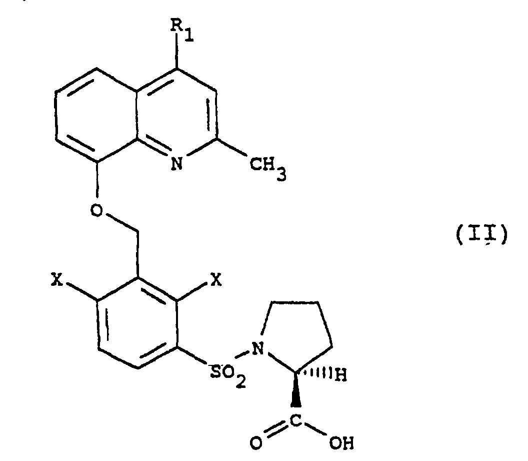 dans laquelle :
   - R₁: représente un atome d'hydrogène, un groupe alkyle en C₁-C₃ à chaîne hydrocarbonée linéaire ou ramifiée ou le groupe trifluorométhyle, et
   - X: représente un halogène ou le groupe méthyle, avec une amine de formule XII : 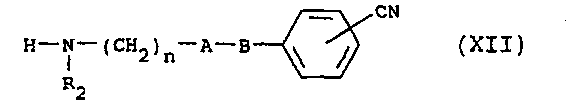
   dans laquelle
   - A: représente un groupe -N(R₃)-CO- ou un groupe -CO-N(R₃)-,
   - B: représente une liaison simple, -CH₂- ou -CH₂-O-,
   - R₂ et R₃: représentent chacun indépendamment H ou un groupe alkyle en C₁-C₃.
   - n: représente 2, 3, 4 ou 5, et
   - W: représente CH ou un atome d'azote,
   dans des conditions analogues à celles décrites précédemment à l'étape (1) du procédé général, pour obtenir un composé de formule IXa: 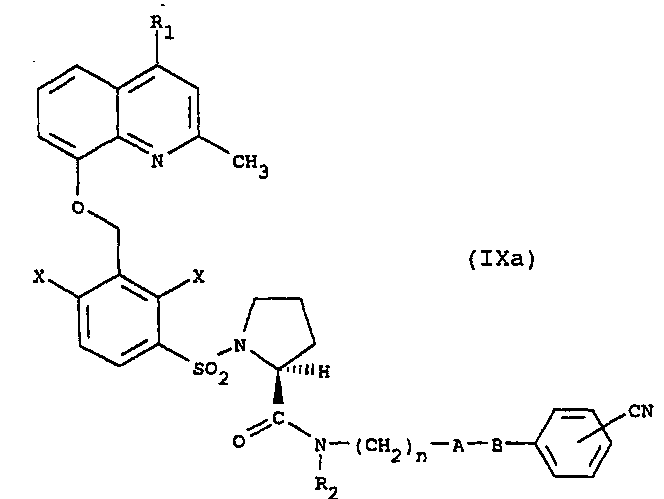 dans laquelle A, B, R₁, R₂, R₃, X, W et n conservent la même signification que dans les produits de départ ; et,
(β) effectuer les étapes analogues à celles décrites précédemment dans la variante B pour obtenir le composé de formule I à partir du composé de formule IXa par transformation du groupe cyano en groupe amidine.

Si nécessaire, le composé de formule I, normalement obtenu sous forme de base libre selon les modes opératoires ci-dessus, peut être transformé en l'un de ses sels avec un acide par action dudit acide généralement en excès et en solution dans un solvant.

L'invention sera mieux comprise à la lecture des exemples de préparation qui suivent et des résultats d'essais pharmacologiques obtenus avec des composés selon l'invention.

Bien entendu l'ensemble de ces éléments n'est pas limitatif mais est donné à titre d'illustration.

Dans le cas de composés présentant dans leur structure un carbone asymétrique, l'absence d'indication particulière ou la mention (R,S) signifie qu'il s'agit de composés racémiques ; dans le cas de composés présentant une chiralité, celle-ci est indiquée à la suite immédiate de l'indexation du substituant porté par ledit carbone asymétrique : on utilise alors les signes (R) ou (S), conformément aux règles de Cahn, Ingold et Prelog. La nomenclature utilisée dans les exemples est celle préconisée par les Chemical Abstracts : ainsi certains dérivés de la L-proline peuvent devenir, après réaction de la fonction acide avec une amine, des dérivés de la 2(S)-pyrrolidinecarboxamide.

Dans la partie expérimentale, les "préparations" sont relatives aux composés intermédiaires et les "exemples" sont relatifs aux produits selon l'invention.

Les points de fusion (F) indiqués ci-après sont en général mesurés à l'aide d'un banc Koffler et ne sont pas corrigés, il s'agit alors de points de fusion instantanée.

Les caractéristiques spectrales des signaux de résonance magnétique nucléaire (RMN) sont données pour le proton (¹H) ou pour l'isotope 13 du carbone (¹³C) : on indique le déplacement chimique par rapport au signal du tétraméthylsilane et, entre parenthèses, la forme du signal (s pour singulet, d pour doublet, t pour triplet, q pour quadruplet, m pour multiplet, sl pour signal large) et le nombre de protons concernés par le signal. A titre indicatif, les spectres RMN ¹H ont été réalisés à 300 MHz.

### Exemple 1

### 1-[[3-[(2,4-diméthylquinolin-8-yl)oxyméthyl]-2,4-dichlorophényl]sulfonyl]-N-[2-[[4-(aminoiminométhyl)phényl]carbonylamino]éthyl]-2(S)-pyrrolidinecarboxamide.

On prépare une solution de 0,15 g (7,25.10⁻⁴ mole) du chlorhydrate de l'acide 4-(aminoiminométhyl)benzoïque dans 10 ml de diméthylformamide (DMF) et on ajoute 0,15 g (7,98.10⁻⁴ mole) de chlorhydrate de 1-(3-diméthylaminopropyl)-3-éthyl-carbodiimide (EDCI) et 0,11 g (7,25.10⁻⁴ mole) de 1-hydroxy-7-azabenzotriazole (HOAT). On agite le mélange résultant pendant 30 minutes à température ambiante (environ 20°C) puis on ajoute goutte-à-goutte une solution de 0,4 g (7,25.10⁻⁴ mole) de 1-[[3-[(2,4-diméthylquinolin-8-yl)oxyméthyl]-2,4-dichlorophényl]sulfonyl]-N-[2-amino-éthyl]-2(S)-pyrrolidine-carboxamide, dans 10 ml de DMF. Le milieu réactionnel est agité pendant 5 heures à température ambiante puis versé sur de l'eau glacée. On ajoute ensuite doucement une solution de soude 1N jusqu'à pH 8 puis on extrait le mélange avec du dichlorométhane. La phase organique est lavée à l'eau, séchée sur sulfate de sodium puis concentrée sous pression réduite. Le produit brut est purifié par chromatographie sur gel de silice greffé NH₂ (LICHROPREP® NH₂) avec pour éluant le mélange dichlorométhane/méthanol (95/5 ; v/v). On obtient ainsi 0,2 g du produit attendu (rendement = 40 %). Le produit est utilisé directement pour obtenir le sel.

### Exemple 2

### 1-[[3-[(2,4-diméthylquinolin-8-yl)oxyméthyl]-2,4-dichlorophényl]sulfonyl]-N-[2-[[4-(aminoiminométhyl)phényl]carbonylamino]éthyl]-2(S)-pyrrolidinecarboxamide, dichlorhydrate

On dissout 0,2 g (0,287.10⁻³ mole) du composé obtenu selon l'exemple 1 dans 2 ml d'une solution d'acide chlorhydrique 1N. La solution obtenue est lyophilisée. Le résidu de lyophilisation est repris en solution dans 2 ml d'eau distillée et lyophilisé à nouveau. On obtient ainsi 220 mg du composé attendu sous forme d'un solide blanc amorphe (rendement quantitatif).
F = 198°C
[α]²⁷_{D} = - 38° (c = 0,31 ; CH₃OH)

### Exemple 3

### 1-[[3-[(2,4-diméthylquinolin-8-yl)oxyméthyl]-2,4-dichlorophényl]sulfonyl]-N-[2-[[2-[4-(aminoiminométhyl)phénoxy]-1-oxoéthyl]amino]éthyl]-2(S)-pyrrolidinecarboxamide

En opérant de façon analogue à l'exemple 1, au départ de chlorhydrate de l'acide 4-(aminoiminométhyl)phénoxyacétique, on obtient le produit attendu sous forme d'un solide blanc (rendement = 31 %).
F = 156-160°C
[α]²³_{D} = - 30° (c = 0,25 ; CH₃OH)

### Exemple 4

### 1-[[3-[(2,4-diméthylquinolin-8-yl)oxyméthyl]-2,4-dichlorophényl]sulfonyl]-N-[2-[[2-[4-(aminoiminométhyl)phénoxy]-1-oxoéthyl]amino]éthyl]-2(S)-pyrrolidinecarboxamide, dichlorhydrate

En opérant de façon analogue à l'exemple 2, au départ du composé obtenu selon l'exemple 3, on obtient le produit attendu (rendement pratiquement quantitatif).
F = 184-188°C
[α]²⁴_{D} = - 27° (c = 0,3 ; CH₃OH)

### Exemple 5

### 1-[[3-[(2,4-diméthylquinolin-8-yl)oxyméthyl]-2,4-dichlorophényl]sulfonyl]-N-[3-[[4-(aminoiminométhyl)phényl]carbonylamino]propyl]-2(S)-pyrrolidinecarboxamide

En opérant de façon analogue à l'exemple 1, au départ du chlorhydrate de 1-[[3-[(2,4-diméthylquinolin-8-yl)oxyméthyl]-2,4-dichlorophényl]sulfonyl]-N-[3-aminopropyl]-2(S)-pyrrolidinecarboxamide, et en présence de N-méthylmorpholine, on obtient le produit attendu (rendement = 48 %).
F = 178-180°C
[α]²⁵_{D} = - 39° (c = 0,32 ; CH₃OH)

### Exemple 6

### 1-[[3[(2,4-diméthylquinolin-8-yl)oxyméthyl]2,4-dichlorophényl]sulfonyl]-N-[3-[[4-(aminoiminométhyl)phényl]carbonylamino]propyl]-2(S)-pyrrolidinecarboxamide, dichlorhydrate

On prépare une solution de 1,3 g (1,82.10⁻³ mole) du composé obtenu selon l'exemple 5 dans 20 ml d'acétate d'éthyle et 6 ml d'éthanol. On ajoute 1,4 ml d'une solution saturée de chlorure d'hydrogène dans l'éther éthylique. Les cristaux formés sont filtrés et lavés à l'éther éthylique, puis remis en solution dans 25 ml d'eau distillée. La solution obtenue est lyophilisée et on obtient ainsi 1,25 g du produit attendu sous forme d'un solide blanc amorphe (rendement = 87,4 %).
F = 200-202°C
[α]²⁵_{D} = - 36° (c = 0,3 ; CH₃OH)

### Exemple 7

### 1-[[3-[(2,4-diméthylquinolin-8-yl)oxyméthyl]-2,4-dichlorophényl]sulfonyl]-N-[3-[[2-[4-(aminoiminométhyl)phényl]-1-oxoéthyl]amino]propyl]-2(S)-pyrrolidinecarboxamide

En opérant de façon analogue à l'exemple 5, au départ du chlorhydrate de l'acide 4-(aminoiminométhyl)phénylacétique, on obtient le produit attendu (rendement = 39 %).
F = 184-188°C
[α]²⁴_{D} = - 30° (c = 0,3 ; CH₃OH)

### Exemple 8

### 1-[[3-[(2,4-diméthylquinolin-8-yl)oxyméthyl]-2,4-dichlorophényl]sulfonyl]-N-[3-[[2-[4-(aminoiminométhyl)phényl]-1-oxoéthyl]amino]propyl]-2(S)-pyrrolidinecarboxamide, dichlorhydrate

On prépare une solution de 120 mg (0,165.10⁻³ mole) du composé obtenu selon l'exemple 7, dans 5 ml d'éthanol et on ajoute 1 ml d'une solution saturée de chlorure d'hydrogène dans l'éther éthylique. Après 30 minutes d'agitation du mélange à température ambiante, on concentre le milieu réactionnel sous pression réduite. Le résidu est repris en solution dans 10 ml d'eau. Après filtration, la solution est lyophilisée. On obtient ainsi 130 mg du produit attendu sous forme d'un solide blanc (rendement = 98 %).
F = 186-190°C
[α]²⁴_{D} = - 25° (c = 0,30 ; CH₃OH)

### Exemple 9

### 1-[[3-[(2,4-diméthylquinolin-8-yl)oxyméthyl]-2,4-dichlorophényl]sulfonyl]-N-[3-[[2-[4-(aminoiminométhyl)phénoxy]-1-oxoéthyl]amino]propyl]-2(S)-pyrrolidinecarboxamide

En opérant de façon analogue à l'exemple 7, au départ du chlorhydrate de l'acide 4-(aminoiminométhyl)phénoxyacétique, on obtient le produit attendu (rendement = 35 %).
F = 124-128°C
[α]²⁴_{D} = - 21° (c = 0,32 ; CHCl₃)

### Exemple 10

### 1-[[3-[(2,4-diméthylquinolin-8-yl)oxyméthyl]-2,4-dichlorophényl]sulfonyl]-N-[3-[[2-[4-(aminoiminométhyl)phénoxy]-1-oxoéthyl]amino]propyl]-2(S)-pyrrolidinecarboxamide, dichlorhydrate

En opérant de façon analogue à l'exemple 6, au départ du composé obtenu selon l'exemple 9, on obtient le produit attendu (rendement = 96 %).
F = 168-172°C
[α]²⁵_{D} = - 20° (c = 0,3 ; CH₃OH)

### PREPARATION I

### 1-[[3-[(2,4-diméthylquinolin-8-yl)oxyméthyl]-2,4-dichlorophényl]sulfonyl]-N-[3-[(3-cyanophényl)carbonylamino]propyl]-2(S)-pyrrolidinecarboxamide

En opérant de façon analogue à l'exemple 5, au départ d'acide 3-cyanobenzoïque, on obtient le produit attendu sous forme d'un solide blanc, après purification par chromatographie sur gel de silice avec pour éluant le mélange dichlorométhane/méthanol (98/2 ; v/v) (rendement = 75 %).
F = 102-106°C
[α]²⁴_{D} = - 40° (c = 0,33 ; CH₃OH)

### PREPARATION II

### 1-[[3-[(2,4-diméthylquinolin-8-yl)oxyméthyl]-2,4-dichlorophényl]sulfonyl]-N-[3-[[3-[(amino)(hydroxyimino)méthyl]phényl]carbonylamino]propyl]-2(S)-pyrrolidinecarboxamide

On prépare une solution de 1,1 g (1,58.10⁻³ mole) du composé obtenu selon la préparation I dans 30 ml de diméthylsulfoxyde et on ajoute 0,55 g (7,9.10⁻³ mole) de chlorhydrate d'hydroxylamine, puis 0,8 g (7,9.10⁻³ mole) de triéthylamine. Après deux heures d'agitation à température ambiante, on ajoute à nouveau les mêmes quantités de ces deux réactifs et on poursuit l'agitation pendant 4 heures. On ajoute ensuite 200 ml d'eau. Le précipité obtenu est séparé par filtration, repris en solution dans du dichlorométhane. La solution organique est lavée à l'eau, séchée sur sulfate de sodium puis concentrée sous pression réduite. On obtient ainsi 1,13 g du produit attendu sous forme de cristaux blancs (rendement = 98 %).
F = 108-110°C
[α]²⁴_{D} = - 30° (c = 0,38 ; CH₃OH)

### PREPARATION III

### 1-[[3-[(2,4-diméthylquinolin-8-yl)oxyméthyl]-2,4-dichlorophényl]sulfonyl]-N-[3-[[3-[(amino)(acétoxyimino)méthyl]phényl]carbonylamino]propyl]-2(S)-pyrrolidinecarboxamide

On ajoute 0,03 g (0,28.10⁻³ mole) d'anhydride acétique à une solution de 0,2 g (0,27.10⁻³ mole) du composé obtenu selon la préparation II dans 10 ml de dichlorométhane anhydre et on agite le mélange réactionnel pendant 15 heures. On ajoute environ 50 ml de dichlorométhane au mélange, on lave cette phase organique à l'eau, puis on sèche sur sulfate de sodium. Après concentration sous pression réduite on obtient 0,20 g du produit attendu sous forme d'un solide blanc (rendement = 95 %).
F = 100-104°C
[α]²⁴_{D} = - 20° (c = 0,29 ; CH₃OH)

### Exemple 11

### 1-[[3-[(2,4-diméthylquinolin-8-yl)oxyméthyl]-2,4-dichlorophényl]sulfonyl]-N-[3-[[3-(aminoiminométhyl)phényl]carbonylamino]propyl]-2(S)-pyrrolidinecarboxamide

On prépare une solution de 0,2 g (0,26.10⁻³ mole) du composé obtenu selon la préparation III dans 5 ml de méthanol et on ajoute 10 mg de catalyseur de Lindlar (à 5 % de palladium). Le mélange est agité sous atmosphère d'hydrogène à pression atmosphérique, à température ambiante, pendant 3 heures. Après élimination du catalyseur par filtration, la solution est concentrée sous pression réduite. Le produit brut obtenu est purifié par chromatographie sur gel de silice greffé NH₂ (LICHROPREP® NH₂), avec pour éluant par le mélange dichlorométhane/méthanol (95/5 ; v/v). On obtient ainsi 0,11 g du produit attendu sous forme d'un solide blanc (rendement = 55 %).
F = 116-120°C
[α]²⁴_{D} = - 36° (c = 0,35 ; CH₃OH)

### Exemple 12

### 1-[[3-[(2,4-diméthylquinolin-8-yl)oxyméthyl]-2,4-dichlorophényl]sulfonyl]-N-[3-[[3-(aminoiminométhyl)phényl]carbonylamino]propyl]-2(S)-pyrrolidinecarboxamide, dichlorhydrate

En opérant de façon analogue à l'exemple 8, au départ du composé obtenu selon l'exemple 11, on obtient le produit attendu (rendement = 98 %).
F = 192-194°C
[α]²²_{D} = - 31° (c = 0,3 ; CH₃OH)

### PREPARATION IV

### Acide N-éthyl-N-(3-amino-propyl)carbamique, 1,1-diméthyléthyl ester

On prépare une solution de 3,42 g (0,017 mole) d'acide N-éthyl-N-(2-cyanoéthyl)carbamique, 1,1-diméthyléthyl ester dans 70 ml d'éthanol et on ajoute 0,5 g de nickel de Raney. Le mélange est agité sous atmosphère d'hydrogène à pression atmosphérique, à température ambiante, pendant 6 heures. On sépare le catalyseur par filtration puis on concentre le filtrat sous pression réduite. L'huile résiduelle est ensuite purifiée par chromatographie sur gel de silice greffé NH₂ avec pour éluant le mélange toluène/isopropanol (95/5 ; v/v). On obtient ainsi 1,72 g du produit attendu sous forme d'huile incolore (rendement = 50 %).
RMN ¹H (300 MHz ; CDCl₃)
1,09 (t,3H) ; 1,28 (d,2H) ; 1,46 (s,9H) ; 1,64 (m,2H) ; 2,67 (t,2H) ; 3,25 (m,4H).

### PREPARATION V

### 1-[[3-[(2,4-diméthylquinolin-8-yl)oxyméthyl]-2,4-dichlorophényl]sulfonyl]-N-[3-[(éthyl)(1,1-diméthyléthoxycarbonyl)amino]propyl]-2(S)-pyrrolidinecarboxamide

On prépare une solution de 3,85 g (7,55.10⁻³ mole) de 1-[[3-[(2,4-diméthylquinolin-8-yl)oxyméthyl]-2,4-dichlorophényl]sulfonyl]-L-proline dans 20 ml de dichlorométhane et on ajoute 1,59 g (8,3.10⁻³ mole) de EDCI, 1,13 g (8,3.10⁻³ mole) de HOAT, puis 1,68 g (8,3.10⁻³ mole) d'acide N-éthyl-N-(3-aminopropyl)carbamique, 1,1-diméthyléthyl ester. Le mélange réactionnel est agité pendant 24 heures à température ambiante puis concentré sous pression réduite. Le résidu est purifié par chromatographie sur gel de silice avec pour éluant un mélange dichlorométhane/méthanol (98/2 ; v/v). On obtient ainsi 5 g du produit attendu sous forme d'un solide amorphe jaune clair (rendement = 95 %).
F = 80°C
[α]²⁵_{D} = - 34° (c = 0,47 ; CH₃OH)

### PREPARATION VI

### 1-[[3-[(2,4-diméthylquinolin-8-yl)oxyméthyl]-2,4-dichlorophényl]sulfonyl]-N-[3-(éthylamino)propyl]-2(S)-pyrrolidinecarboxamide

On ajoute 3 ml d'acide trifluoroacétique et 0,408 g (3,78.10⁻³ mole) d'anisole à une solution, préalablement refroidie à 0°C, de 2,62 g (3,78.10⁻³ mole) du composé obtenu selon la préparation V dans 8 ml de dichlorométhane. Le mélange réactionnel est agité 1 heure à 0°C puis 20 heures à température ambiante. Après élimination du solvant sous pression réduite, le résidu est additionné d'eau et la phase aqueuse est amenée à pH basique à l'aide d'une solution de NaOH 1N. Après extraction à l'acétate d'éthyle, la phase organique est lavée à l'eau, séchée sur sulfate de sodium et concentrée sous pression réduite. On obtient ainsi 2,14 g du produit attendu sous forme d'un solide beige (rendement = 95 %).
F = 70°C
[α]²⁵_{D} = - 44° (c = 0,38 ; CH₃OH)

### Exemple 13

### 1-[[3-[(2,4-diméthylquinolin-8-yl)oxyméthyl]-2,4-dichlorophényl]sulfonyl]-N-[3-[[éthyl]-[[4-(aminoiminométhyl)phényl]carbonyl]amino]propyl]-2(S)-pyrrolidinecarboxamide

On prépare une solution de 0,2 g (0,337.10⁻³ mole) du composé obtenu selon la préparation VI dans 10 ml de dichlorométhane, et on ajoute à la solution 0,07 g (0,337.10⁻³ mole) du chlorhydrate du chlorure de l'acide 4-(aminoiminométhyl)benzoïque et 0,027 g (0,27.10⁻³ mole) de N-méthylmorpholine. Le mélange réactionnel est agité pendant 20 heures puis concentré sous pression réduite. Le résidu est repris en solution dans l'eau et la solution est amenée à pH légèrement alcalin à l'aide de NaOH 1N. On extrait avec du dichlorométhane. La phase organique est lavée à l'eau, séchée sur sulfate de sodium et concentrée sous pression réduite. Le résidu est purifié par chromatographie sur silice greffé NH₂ avec pour éluant le mélange dichlorométhane-méthanol (95/5 ; v/v). On obtient ainsi 0,12 g du produit attendu sous forme d'un solide blanc-crème (rendement = 48 %).
F = 160°C

### Exemple 14

### 1-[[3-[(2,4-diméthylquinolin-8-yl)oxyméthyl]-2,4-dichlorophényl]sulfonyl]-N-[3-[[éthyl]-[[4-(aminoiminométhyl)phényl]carbonyl]amino]propyl]-2(S)-pyrrolidinecarboxamide, dichlorhydrate

En opérant de façon analogue à l'exemple 8, au départ du composé obtenu selon l'exemple 13, on obtient le produit attendu (rendement = 80 %).
F = 193°C
[α]²⁵_{D} = - 27° (c = 0,37 ; CH₃OH)

### PREPARATION VII

### 1-[[3-[(2,4-diméthylquinolin-8-yl)oxyméthyl]-2,4-dichlorophényl]sulfonyl]-N-[3-[(méthyl)(1,1-diméthyléthoxycarbonyl)amino]propyl]-2(S)-pyrrolidinecarboxamide

En opérant de façon analogue à la préparation V, au départ d'acide N-méthyl-N-(3-amino-propyl)carbamique, 1,1-diméthyléthyl ester, on obtient le produit (rendement = 73 %).
F = 80°C
[α]²⁵_{D} = - 40° (c = 0,42 ; CH₃OH)

### PREPARATION VIII

### 1-[[3-[(2,4-diméthylquinolin-8-yl)oxyméthyl]-2,4-dichlorophényl]sulfonyl]-N-[3-(méthylamino)propyl]-2(S)-pyrrolidinecarboxamide

En opérant de façon analogue à la préparation VI, au départ du composé obtenu selon la préparation VII, on obtient le produit attendu (rendement = 92 %).
F = 85°C
[α]²⁵_{D} = - 37° (c = 0,39 ; CH₃OH)

### Exemple 15

### 1-[[3-[(2,4-diméthylquinolin-8-yl)oxyméthyl]-2,4-dichlorophényl]sulfonyl]-N-[3-[[méthyl]-[[4-(aminoiminométhyl)phényl]carbonyl]amino]propyl]-2(S)-pyrrolidinecarboxamide

En opérant de façon analogue à l'exemple 13, au départ du composé obtenu selon la préparation VIII, on obtient le produit attendu (rendement = 51 %).
F = 158°C
[α]²⁵_{D} = - 29° (c = 0,55 ; CH₃OH)

### Exemple 16

### 1-[[3-[(2,4-diméthylquinolin-8-yl)oxyméthyl]-2,4-dichlorophényl]sulfonyl]-N-[3-[[méthyl]-[[4-(aminoiminométhyl)phényl]carbonyl]amino]propyl]-2(S)-pyrrolidinecarboxamide, dichlorhydrate

En opérant de façon analogue à l'exemple 8, au départ du composé obtenu selon l'exemple 15, on obtient le produit attendu (rendement = 76 %).
F = 196°C
[α]²⁵_{D} = - 29° (c = 0,36 ; CH₃OH)

### PREPARATION IX

### 1-[[3-[(2-méthylquinolin-8-yl)oxyméthyl]-2,4-dichlorophényl]sulfonyl]-N-[3-[(4-cyanophényl)carbonylamino]propyl]-2(S)-pyrrolidinecarboxamide

En opérant de façon analogue à l'exemple 5, au départ d'acide 4-cyanobenzoïque, et du chlorhydrate de 1-[[3-[(2-méthyl-quino]in-8-yl)oxyméthyl]-2,4-dichlorophényl]sulfonyl]-N-[3-amino-propyl]-2(S)-pyrrolidinecarboxamide, et après purification par chromatographie sur gel de silice avec pour éluant un mélange dichlorométhane/méthanol (98/2 ; v/v), on obtient le produit attendu (rendement = 75 %).
F = 78-80°C
[α]²⁴_{D} = - 39° (c = 0,3 ; CH₃OH)

### PREPARATION X

### 1-[[3-[(2-méthylquinolin-8-yl)oxyméthyl]-2,4-dichlorophényl]sulfonyl]-N-[3-[[4-[(amino)(hydroxyimino)méthyl]phényl]carbonylamino]propyl]-2(S)-pyrrolidinecarboxamide

En opérant de façon analogue à la préparation II, au départ du composé obtenu selon la préparation IX, on obtient le produit attendu (rendement = 70 %).
F = 120-124°C
[α]²⁴_{D} = - 35° (c = 0,3 ; CH₃OH)

### PREPARATION XI

### 1-[[3-[(2-méthylquinolin-8-yl)oxyméthyl]-2,4-dichlorophényl]sulfonyl]-N-[3-[[4-[(amino)(acétoxyimino)méthyl]phényl]carbonylamino]propyl]-2(S)-pyrrolidinecarboxamide

En opérant de façon analogue à la préparation III, au départ du composé obtenu selon la préparation X, on obtient le produit attendu (rendement = 72 %).
F = 122-126°C
[α]²⁴_{D} = - 36° (c = 0,27 ; CH₃OH)

### Exemple 17

### 1-[[3-[(2-méthylquinolin-8-yl)oxyméthyl]-2,4-dichlorophényl]sulfonyl]-N-[3-[[4-(aminoiminométhyl)phényl]carbonylamino]propyl]-2(S)-pyrrolidinecarboxamide

En opérant de façon analogue à l'exemple 11, au départ du composé obtenu selon la préparation XI, on obtient le produit attendu (rendement = 76 %).
F = 118-120°C
[α]²⁴_{D} = - 38° (c = 0,35 ; CH₃OH)

### Exemple 18

### 1-[[3-[(2-méthylquinolin-8-yl)oxyméthyl]-2,4-dichlorophényl]sulfonyl]-N-[3-[[4-(aminoiminométhyl)phényl]carbonylamino]propyl]-2(S)-pyrrolidinecarboxa-mide, dichlorhydrate

En opérant de façon analogue à l'exemple 8, au départ du composé obtenu selon l'exemple 17, on obtient le produit attendu (rendement = 97 %).
F = 186-188°C
[α]²³_{D} = - 37° (c = 0,3 ; CH₃OH)

### Exemple 19

### 1-[[3-[(2,4-diméthylquinolin-8-yl)oxyméthyl]-2,4-dichlorophényl]sulfonyl]-N-[4-[[4-(aminoiminométhyl)phényl]carbonylamino]butyl]-2(S)-pyrrolidinecarboxamide, dichlorhydrate

On prépare une solution de 0,34 g (1,69.10⁻³ mole) de chlorhydrate de l'acide 4-(aminoiminométhyl)benzoïque dans 5 ml de diméthylformamide (DMF) et on ajoute 0,46 g (1,69.10⁻³ mole) de EDCI et 0,32 g (1,69.10⁻³ mole) de HOAT. On agite le mélange pendant 30 minutes à température ambiante. La solution obtenue est ensuite ajoutée goutte-à-goutte à une solution de 1 g (1,53.10⁻³ mole) de 1-[[3-[(2,4-diméthylquinolin-8-yl)oxyméthyl]-2,4-dichlorophényl]sulfonyl]-N-[4-aminobutyl]-2(S)-pyrrolidinecarboxamide (sous forme de dichlorhydrate) dans 5 ml de DMF et 0,46 g (4,6.10⁻³ mole) de triéthylamine. Le mélange réactionnel est agité pendant 4 heures à température ambiante puis versé sur de l'eau glacée. On ajoute lentement une solution de NaOH 1N de façon à amener le pH à 8 et on extrait plusieurs fois avec du dichlorométhane. Les phases organiques rassemblées sont lavées à l'eau, séchées sur sulfate de sodium puis concentrées sous pression réduite. Le produit brut est purifié par chromatographie une première fois sur gel de silice greffé NH₂ en éluant par un mélange dichlorométhane/méthanol (98/2 ; v/v), une seconde fois sur gel de silice greffé C18 (type RP18) avec pour éluant le mélange dichlorométhane/HCl gaz 0,2 N dans méthanol (98/2 ; v/v). Après élimination des solvants sous pression réduite, le produit est mis en solution dans 5 ml d'eau distillée et la solution est lyophilisée. On obtient ainsi 150mg du produit attendu sous forme d'un solide jaune pâle (rendement = 13 %).
F = 196-200°C
[α]²⁶_{D} = - 20° (c = 0,285 ; CH₃OH)

### PREPARATION XII

### 1-[[3-[(2,4-diméthylquinolin-8-yl)oxyméthyl]-2,4-dichlorophényl]sulfonyl]-N-[5-[(1,1-diméthyléthoxycarbonyl)amino]pentyl]-2(S)-pyrrolidinecarboxamide

En opérant de façon analogue à la préparation V, au départ de l'acide (5-aminopentyl)-carbamique,1,1-diméthyléthyl ester, on obtient le produit attendu (rendement = 43 %).
F = 78-82°C

### PREPARATION XIII

### 1-[[3-[(2,4-diméthylquinolin-8-yl)oxyméthyl]-2,4-dichlorophényl]sulfonyl]-N-(5-aminopentyl)-2(S)-pyrrolidinecarboxamide, dichlorhydrate

On ajoute 2,9 g (4,2.10⁻³ mole) du composé obtenu selon la préparation XII à 25 ml d'une solution d'acide chlorhydrique 1N et on agite le mélange à 50°C pendant 3 heures. Le milieu réactionnel est ensuite refroidi à 15°C, lavé avec de l'acétate d'éthyle. La phase aqueuse est concentrée sous pression réduite puis reprise avec 50 ml d'eau distillée. La solution est filtrée puis lyophilisée. On obtient ainsi le produit attendu sous forme d'un solide blanc (rendement = 90 %).
F = 162-166°C
[α]²⁴_{D} = - 29° (c = 0,31 ; CH₃OH)

### Exemple 20

### 1-[[3-[(2,4-diméthylquinolin-8-yl)oxyméthyl]-2,4-dichlorophényl]sulfonyl]-N-[5-[[4-(aminoiminométhyl)phényl]carbonylamino]pentyl]-2(S)-pyrrolidinecarboxamide

En opérant de façon analogue à l'exemple 1, au départ du composé obtenu selon la préparation XIII, on obtient le produit attendu (rendement = 30 %).
F = 150-152°C
[α]²⁴_{D} = - 14° (c = 0,32 ; CH₃OH)

### Exemple 21

### 1-[[3-[(2,4-diméthylquinolin-8-yl)oxyméthyl]-2,4-dichlorophényl]sulfonyl]-N-[5-[[4-(aminoiminométhyl)phényl]carbonylamino]pentyl]-2(S)-pyrrolidinecarboxamide, dichlorhydrate

En opérant de façon analogue à l'exemple 8, au départ du composé obtenu selon l'exemple 20, on obtient le produit attendu (rendement = 97 %).
F = 182-185°C
[α]²⁵_{D} = - 15° (c = 0,31 ; CH₃OH)

### PREPARATION XIV

### Acide N-(méthyl)-N-[3-[(4-cyanophényl)carbonylamino]propyl]carbamique, 1,1-diméthyléthyl ester

On prépare une solution de 1,23 g (6,53.10⁻³ mole) d'acide N-méthyl-N-[3-aminopropyl]carbamique, 1,1-diméthyléthyl ester dans 10 ml de dichlorométhane et 5 ml de pyridine et on ajoute 1,08 g (6,53.10⁻³ mole) de chlorure de l'acide 4-cyanobenzoïque. Après 20 heures sous agitation à température ambiante, on ajoute du dichlorométhane et on lave cette phase organique à l'eau, puis à l'aide d'une solution d'acide chlorhydrique 1N, puis à nouveau à l'eau. Après séchage sur sulfate de magnésium, la phase organique est concentrée sous pression réduite, le résidu obtenu est purifié par chromatographie sur gel de silice avec pour éluant le mélange toluène/acétate d'éthyle 8/2 (v/v). On obtient ainsi 1 g du produit attendu sous forme d'un solide blanc crème (rendement = 48 %).
F < 50°C

### PREPARATION XV

### 4-cyano-N-[3-(méthylamino)propyl]benzamide

En opérant de façon analogue à la préparation VI, au départ du composé obtenu selon la préparation XIV, on obtient le produit attendu sous forme d'une huile jaune clair (rendement = 89 %).
RMN ¹H (DMSO)
1,70 (q,2H) ; 2,34 (s,3H) ; 2,61 (t,3H) ; 3,3 (m;3H) ; 7,97 (s,4H) ; 8,83 (s1,1H).

### PREPARATION XVI

### 1-[[3-[(2,4-diméthylquinolin-8-yl)oxyméthyl]-2,4-dichlorophényl]sulfonyl]-N-[méthyl]-N-[3-[(4-cyanophényl)carbonylamino]propyl]-2(S)-pyrrolidinecarboxamide

En opérant de façon analogue à la préparation V, au départ du composé obtenu selon la préparation XV, on obtient le produit attendu (rendement = 40 %).
F = 100°C
[α]¹⁹_{D} = - 30° (c = 0,32 ; CH₃OH)

### PREPARATION XVII

### 1-[[3-[(2,4-diméthylquinolin-8-yl)oxyméthyl]-2,4-dichlorophényl]sulfonyl]-N-[méthyl]-N-[3-[[4-[amino-(hydroxyimino)méthyl]phényl]carbonylamino]-propyl]-2(S)-pyrrolidinecarboxamide

En opérant de façon analogue à la préparation II, au départ du composé obtenu à la préparation XVI, on obtient le produit attendu (rendement
= 95 %).
F = 65-70°C
[α]¹⁸_{D} = + 18° (c = 0,25 ; CH₃OH)

### PREPARATION XVIII

### 1-[[3-[(2,4-diméthylquinolin-8-yl)oxyméthyl]-2,4-dichlorophényl]sulfonyl]-N-[méthyl]-N-[3-[[4-[amino-(acétoxyimino)méthyl]phényl]carbonylamino]-propyl]-2(S)-pyrrolidinecarboxamide

En opérant de façon analogue à la préparation III, au départ du composé obtenu à la préparation XVII, on obtient le produit attendu (rendement = 87 %).
F = 70°C
[α]¹⁹_{D} = - 4,3° (c = 0,32 ; CH₃OH)

### Exemple 22

### 1-[[3-[(2,4-diméthylquinolin-8-yl)oxyméthyl]-2,4-dichlorophényl]sulfonyl]-N-[méthyl]-N-[3-[[4-(aminoiminométhyl)phényl]carbonylamino]propyl] 2 (S)-pyrrolidinecarboxamide

En opérant de façon analogue à l'exemple 11, au départ du composé obtenu à la préparation XVIII, on obtient le produit attendu (rendement = 73 %).
F = 133°C
[α] ¹⁹_{D} = - 40° (c = 0,33 ; CH₃OH)

### Exemple 23

### 1-[[3-[(2,4-diméthylquinolin-8-yl)oxyméthyl]-2,4-dichlorophényl]sulfonyl]-N-[méthyl]-N-[3-[[4-(aminoiminométhyl)phényl]carbonylamino]propyl]-2(S)-pyrrolidinecarboxamide, dichlorhydrate

On prépare une solution de 0,15 g (0,20.10⁻³ mole) du composé obtenu selon l'exemple 22 dans 5 ml de méthanol anhydre et on ajoute 1 ml d'éther éthylique saturé par du chlorure d'hydrogène. On agite 10 minutes puis on ajoute 20 ml d'éther éthylique. Le précipité obtenu est filtré, rincé à l'éther et redissous dans 5 ml d'eau distillée. Après lyophilisation de la solution, on obtient 0,15 g du produit attendu sous forme d'un solide blanc jaune (rendement = 91 %).
F = 211°C
[α]¹⁹_{D} = - 8° (c = 0,32 ; CH₃OH)

### PREPARATION XIX

### 1-[[3-[(2,4-diméthylquinolin-8-yl)oxyméthyl]-2,4-dichlorophényl]sulfonyl]-N-[méthyl]-N-[3-[(méthyl)(1,1-diméthyléthoxycarbonyl)amino]propyl]-2(S)-pyrrolidinecarboxamide

En opérant de façon analogue à la préparation VII, au départ d'acide N-méthyl-N-[3-(méthylamino)propyl]carbamique, 1,1-diméthyléthyl ester, on obtient le produit attendu (rendement = 78 %).
F = 50°C
[α]²¹_{D} = - 17° (c = 0,38 ; CH₃OH)

### PREPARATION XX

### 1-[[3-[(2,4-diméthylquinolin-8-yl)oxyméthyl]-2,4-dichlorophényl]sulfonyl]-N-[méthyl]-N-[3-(méthylamino)propyl]-2(S)-pyrrolidinecarboxamide

En opérant de façon analogue à la préparation VIII, au départ du composé obtenu selon la préparation XIX, on obtient le produit attendu (rendement = 99 %).
F = 150°C
[α]²¹_{D} = - 26° (c = 0,33 ; CH₃OH)

### PREPARATION XXI

### 1-[[3-[(2,4-diméthylquinolin-8-yl)oxyméthyl]-2,4-dichlorophényl]sulfonyl]-N-[méthyl]-N-[3-[(méthyl)[(4-cyanophényl)carbonyl]amino]propyl]-2(S)-pyrro-lidinecarboxaimde

En opérant de façon analogue à la préparation I, au départ du composé obtenu selon la préparation XX et d'acide 4-cyanobenzoïque, on obtient le produit attendu (rendement = 90 %).
F = 60°C
[α]²¹_{D} = + 6° (c = 0,72 ; CH₃OH)

### PREPARATION XXII

### 1-[[3-[(2,4-diméthylquinolin-8-yl)oxyméthyl]-2,4-dichlorophényl]sulfonyl]-N-[méthyl]-N-[3-[(méthyl)[[4-[amino-(hydroxyimino)méthyl]phényl]-carbonyl]amino]propyl]-2(S)-pyrrolidinecarboxamide

En opérant de façon analogue à la préparation II, au départ du composé obtenu à la préparation XXI, on obtient le produit attendu sous forme d'un produit huileux (rendement = 77 %).
n²³_{D} = 1,571
[α]²¹_{D} = - 18° (c = 0,55 ; CH₃OH)

### PREPARATION XXIII

### 1-[[3-[(2,4-diméthylquinolin-8-yl)oxyméthyl)-2,4-dichlorophényl]sulfonyl]-N-[méthyl]-N-[3-[(méthyl)[[4-[amino-(acétoxyimino)méthyl]phényl]-carbonyl]amino]propyl]-2(S)-pyrrolidinecarboxamide

En opérant de façon analogue à la préparation III, au départ du composé obtenu à la préparation XXII, on obtient le produit attendu (rendement = 60 %).
F = 70°C
[α]²¹_{D} = - 12° (c = 0,45 ; CH₃OH)

### Exemple 24

### 1-[[3-[(2,4-diméthylquinolin-8-yl)oxyméthyl]-2,4-dichlorophényl]sulfonyl]-N-[méthyl]-N-[3-[(méthyl)[[4-(aminoiminométhyl)phényl]carbonyl]amino]-propyl]-2(S)-pyrrolidinecarboxamide

En opérant de façon analogue à l'exemple 11, au départ du composé obtenu à la préparation XXIII, on obtient le produit attendu (rendement = 72 %).
F = 170°C
[α]²¹_{D} = - 16,2° (c = 0,69 ; CH₃OH)

### Exemple 25

### 1-[[3-[(2,4-diméthylquinolin-8-yl)oxyméthyl]-2,4-dichlorophényl]sulfonyl]-N-[méthyl]-N-[3-[(méthyl)[[4-(aminoiminométhyl)-phényl]carbonyl]amino]-propyl]-2(S)-pyrrolidinecarboxamide, dichlorhydrate

En opérant de façon analogue à l'exemple 8, au départ du composé obtenu à l'exemple 22, on obtient le produit attendu (rendement = 86 %).
F = 175°C
[α]²¹_{D} = - 2,4° (c = 0,38 ; CH₃OH)

### PREPARATION XXIV

### 1-[[3-[(2,4-diméthylquinolin-8-yl)oxyméthyl]-2,4-dichlorophényl]sulfonyl]-N-[3-[(5-cyanopyridin-2-yl)carbonylamino]propyl]-2(S)-pyrrolidinecarboxamide

On prépare une suspension de 1,90 g (10,3.10⁻³ mole) de chlorhydrate de l'acide 5-cyanopicolinique dans 50 ml de dichlorométhane et on ajoute 1,10 ml (34.10⁻³ mole) de N-méthylmorpholine. Après 30 mn sous agitation, on ajoute 1,68 g (12,3.10⁻³ mole) de HOAT, puis 2,37 g (12,3.10⁻³ mole) de EDCI. On agite 1 heure à température ambiante puis on ajoute goutte-à-goutte 6,65 g (10,3.10⁻³ mole) du chlorhydrate de 1-[[3-[(2,4-diméthyl-quinolin-8-yl)oxyméthyl]-2,4-dichlorophényl]sulfonyl]-N-[3-aminopropyl]-2(S)-pyrrolidinecarboxamide, et 2,55 ml (72. 10⁻³ mole) de N-méthylmorpholine en solution dans 30 ml de dichlorométhane. Après une nuit sous agitation à température ambiante, le mélange réactionnel est versé sur 100 ml d'eau puis extrait 2 fois par du dichlorométhane. Les phases organiques rassemblées sont lavées à l'eau, séchées sur sulfate de magnésium et concentrées sous pression réduite. Le produit brut obtenu (3,16 g) est purifié par chromatographie sur gel de silice avec pour éluant l'acétate d'éthyle. On obtient ainsi 2,18 g du produit attendu sous forme de cristaux jaune clair (rendement = 30,4 %).
F = 91 - 93°C
[α]²⁰_{D} = - 39 ° (c = 0,99 ; CH₃OH)

### PREPARATION XXV

### 1-[[3-[(2,4-diméthylquinolin-8-yl)oxyméthyl]-2,4-dichlorophényl]sulfonyl]-N-[3-[[5-[(amino)(hydroxyimino)méthyl]pyridin-2-yl]carbonylamino]-propyl]-2(S)-pyrrolidinecarboxamide

En opérant de façon analogue à la préparation II, au départ du composé obtenu selon la préparation XXIV, on obtient le produit attendu sous forme de cristaux jaune pâle (rendement = 72 %).
F = 134 - 136°C
[α]²¹_{D} = - 32° (c = 1,00 ; CH₃OH)

### PREPARATION XXVI

### 1-[[3-[(2,4-diméthylquinolin-8-yl)oxyméthyl]-2,4-dichlorophényl]sulfonyl]-N-[3-[[5-[(amino)(acétoxyimino)méthyl]-pyridin-2-yl]carbonylamino]-propyl]-2(S)-pyrrolidinecarboxamide

En opérant de façon analogue à la préparation III au départ du composé obtenu selon la préparation XXV, on obtient le produit attendu sous forme de cristaux blancs crème (rendement = 92 %).
F = 116-118°C
[α]²²_{D} = - 13° (c = 1,05 ; CH₃OH)

### Exemple 26

### 1-[[3-[(2,4-diméthylquinolin-8-yl)oxyméthyl]-2,4-dichlorophényl]sulfonyl]-N-[3-[[5-(aminoiminométhyl)pyridin-2-yl]carbonylamino]propyl]-2(S)-pyrro-lidinecarboxamide

En opérant de façon analogue à l'exemple 11, au départ du composé obtenu selon la préparation XXVI, on obtient le produit attendu sous forme de cristaux blancs (rendement = 65 %).
F = 145-147°C
[α]²²_{D} = - 36° (c = 1,03 ; CH₃OH)

### Exemple 27

### 1-[[3-[(2,4-diméthylquinolin-8-yl)oxyméthyl]-2,4-dichlorophényl]sulfonyl]-N-[3-[[5-(aminoiminométhyl)pyridin-2-yl]carbonylamino]propyl]-2(S)-pyrro-lidinecarboxamide, di(méthanesulfonate)

On prépare une solution de 0,64 g (0,9. 10⁻³ mole) du composé obtenu selon l'exemple 26 dans 12,5 ml de méthanol et on ajoute 86,3 mg d'acide méthanesulfonique. Le mélange est agité pendant une heure à température ambiante puis versé sur 700 ml d'éther éthylique sous agitation. Le précipité obtenu est filtré, lavé avec de l'éther, puis repris en solution dans 60 ml d'eau. La solution obtenue est filtrée et lyophilisée. On obtient ainsi 0,675 g du produit attendu sous forme de flocons blancs (rendement = 92 %).
F = 160°C (décomposition)
[α]²¹_{D} = - 21° (c = 1,03 ; CH₃OH)

### PREPARATION XXVII

### acide 3-[[1-[[3-[(2,4-diméthylquinolin-8-yl)oxyméthyl]-2,4-dichlorophényl]-sulfonyl]pyrrolidin-2-yl]carbonylamino]propanoïque, méthyl ester

On prépare un mélange de 6,85 g (13,4.10⁻³ mole) de N-[[3-[(2,4-diméthylquinolin-8-yl)oxyméthyl]-2,4-dichlorophényl]sulfonyl]-L-proline, 2,01 g (14,7.10⁻³ mole) de HOAT et 2,83 g (14,7.10⁻³ mole) de EDCI dans 50 ml de diméthylformamide. Après 15 mn sous agitation à température ambiante, ce mélange est ajouté goutte-à-goutte à une solution de 2,06 g de 4-aminobutyrate de méthyle (sous forme de chlorhydrate) dans 35 ml de diméthylformamide et 1,48 ml (13,4.10⁻³ mole) de N-méthylmorpholine. Le mélange réactionnel est agité pendant 20 heures à température ambiante puis on ajoute 500 ml de dichlorométhane puis 300 ml d'eau. Après décantation, la phase organique est à nouveau lavée 2 fois avec de l'eau, séchée sur sulfate de sodium puis concentrée sous pression réduite. Le produit brut obtenu est purifié par chromatographie sur gel de silice avec pour éluant un mélange cyclohexane/acétate d'éthyle (3/7 ; v/v). On obtient ainsi 5,3 g du produit attendu sous forme de cristaux jaunes (rendement = 64,5 %).
F = 64 - 67°C
[α]²⁴_{D} = - 40,4° (c = 1,10 ; CH₂Cl₂)

### PREPARATION XXVIII

### acide 3-[[1-[[3-[(2,4-diméthylquinolin-8-yl)oxyméthyl]-2,4-dichlorophényl]-sulfonyl]pyrrolidin-2-yl]carbonylamino]propanoïque, chlorhydrate

On chauffe à 90-100°C pendant 1 heure un mélange de 5,07 g du composé obtenu selon la préparation XXVII avec 9,1 ml d'une solution 1N d'hydroxyde de sodium dans l'eau. Après refroidissement, on ajoute 30 ml d'eau et la phase aqueuse est extraite 2 fois par 50 ml d'acétate d'éthyle, puis acidifiée jusqu'à pH 1 avec une solution 1N d'acide chlorhydrique. Le produit insoluble est repris en solution dans du dichlorométhane et la phase organique est lavée avec un peu d'eau, séchée sur sulfate de sodium, et concentrée sous pression réduite. On obtient ainsi 2,8 g du produit attendu sous forme de cristaux blancs (rendement = 53 %).
F = 146 - 148°C
[α]²⁴_{D} = - 21,2° (c = 1,00 ; CH₃OH)

### Exemple 28

### 1-[[3-[(2,4-diméthylquinolin-8-yl)oxyméthyl]-2,4-dichlorophényl]sulfonyl]-N-t3-[[4-(aminoiminométhyl)phényl]méthylaminocarbonyl]propyl]-2(S)-pyrrolidinecarboxamide

On prépare une solution de 2 g (1,58.10⁻³ mole) de l'acide obtenu selon la préparation XXVIII dans 20 ml de diméthylformamide, et on ajoute 0,364 g (1,89.10⁻³ mole) de EDCI et 0,723 g (1,89.10⁻³ mole) de HATU [O-(7-azabenzotriazol-1-yl)-1,1,3,3-tétraméthyluroniumhexafluorophosphate]. Après 30 mn sous agitation, on ajoute 0,52 ml (4,7.10⁻³ mole) de N-méthylmorpholine, puis 0,352 g (1,58.10⁻³ mole) de dichlorhydrate de 4-(aminoiminométhyl)phénylméthanamine. Le mélange réactionnel est maintenu pendant 16 heures sous agitation à température ambiante puis on ajoute 150 ml d'éther éthylique. Le précipité formé est filtré, lavé à l'eau puis par 10 ml d'une solution 1N d'hydroxyde de sodium, puis à nouveau par de l'eau et séché dans un dessiccateur. Le produit brut ainsi obtenu est purifié par chromatographie sur gel de silice greffé NH₂ avec pour éluant un mélange dichlorométhane/éthanol (95/5 ; v/v). On obtient ainsi 0,21 g du produit attendu sous forme de cristaux blancs (rendement = 18 %).
F = 112 - 114°C
[α]²³_{D} = - 24° (c = 0,64 ; CHCl₃)

### Exemple 29

### 1-[[3-[(2,4-diméthylquinolin-8-yl)oxyméthyl]-2,4-dichlorophényl]sulfonyl]-N-[3-[[4-(aminoiminométhyl)phényl]méthylaminocarbonyl]propyl]-2(S)-pyrrolidinecarboxamide, chlorhydrate

On prépare une solution de 0,169 g (0,23.10⁻³ mole) du composé obtenu selon l'exemple 28, dans 8 ml de dichlorométhane et on ajoute 0,23 ml d'une solution à 1 mole/l de chlorure d'hydrogène dans l'éther éthylique. Après une heure sous agitation à température ambiante, le mélange est concentré sous pression réduite. Le résidu est repris dans de l'éther éthylique et filtré. Le solide obtenu est lavé avec de l'éther puis redissous dans 20 ml d'eau. La solution obtenue est filtrée et lyophilisée. On obtient ainsi 0,16 g du produit attendu sous forme de cristaux blancs (rendement = 90 %).
F = 178 - 180°C
[α]²⁷_{D} = - 3,5° (c = 0,96 ; C₂H₅OH)

### Exemple 30

### 1-[[3-[(2,4-diméthylquinolin-8-yl)oxyméthyl]-2,4-dichlorophényl]sulfonyl]-N-[3-[[4-(aminoiminométhyl)phényl]aminocarbonyl]propyl]-2(S)-pyrrolidinecarboxamide

En opérant de façon analogue à l'exemple 28, au départ de l'acide obtenu selon la préparation XXVIII et de 4-(aminoiminométhyl)aniline, on obtient le produit attendu sous forme de cristaux blancs (rendement = 10 %).
F = 140-141°C

### Exemple 31

### 1-[[3-[(2,4-diméthylquinolin-8-yl)oxyméthyl]-2,4-dichlorophényl]sulfonyl]-N-[3-[[4-(aminoiminométhyl)phényl]aminocarbonyl]propyl]-2(S)-pyrrolidinecarboxamide, chlorhydrate

En opérant de façon analogue à l'exemple 29, au départ du composé obtenu selon l'exemple 30, on obtient le produit attendu sous forme de cristaux blancs (rendement = 90 %).
F = 192-193°C

### PREPARATION XXIX

### 4-[(2-méthoxyphényl)imino]-1,1,1-trifluoro-2-pentanone

On prépare un mélange de 28,4 g (0,23 mole) de 2-méthoxyaniline et de 43,2 g (0,28 mole) de 1,1,1-trifluoro-2,4-pentanedione, que l'on porte à 100-105°C pendant 1 h sous agitation. Après refroidissement, le mélange réactionnel est repris en solution dans l'éther éthylique, la solution obtenue est séchée sur sulfate de sodium puis concentrée sous pression réduite. On obtient ainsi 59,1 g du produit attendu sous forme d'un solide beige (rendement = 99 %).
F = 40°C

### PREPARATION XXX

### 8-méthoxy-2-méthyl-4-(trifluorométhyl)quinoléine

On mélange 10 g (38.10-3 mole) du composé obtenu selon la préparation XXIX avec 50 g d'acide polyphosphorique et on chauffe à 165°C sous agitation pendant 3 heures. Après refroidissement, le milieu réactionnel est versé sur de l'eau glacée. Le mélange obtenu est extrait par de l'éther éthylique et la phase organique est lavée par une solution de bicarbonate de sodium, par une solution diluée d'acide chlorhydrique puis à l'eau. Après sédiage de la phase organique et concentration sous pression réduite, on obtient 5,2 g du produit attendu sous forme d'un solide crème (rendement = 56 %).
F = 112-113°C

### PREPARATION XXXI

### 8-hydroxy-2-méthyl-4-(trifluorométhyl)quinoléine

On refroidit à - 60°C une solution de 6,15 g (25,5.10⁻³ mole) du composé obtenu selon la préparation XXX dans 200 ml de dichlorométhane et on ajoute goutte-à-goutte sous agitation, 130 ml d'une solution 1M de tribromure de bore dans le dichlorométhane. On laisse le milieu réactionnel revenir à température ambiante et on agite ainsi pendant 12 heures. On refroidit le mélange à - 60°C et on ajoute doucement 140 ml de méthanol. On laisse ensuite revenir à température ambiante et on agite ainsi pendant 2 heures. Le mélange est ensuite concentré sous pression réduite et le résidu est repris par du dichlorométhane. La phase organique est lavée par une solution de bicarbonate de sodium puis par de l'eau, puis séchée sur sulfate de sodium et concentrée sous pression réduite. On obtient ainsi 5,60 g du produit attendu sous forme d'un solide jaune (rendement = 96 %).
F = 60-61°C

### PREPARATION XXXII

### N-[[3-[[2-méthyl-4-(trifluorométhyl)quinolin-8-yl]oxyméthyl]-2,4-dichlorophényl]sulfonyl]-L-proline, méthyl ester

On prépare une solution de 1,08 g (4,8.10⁻³ mole) du composé obtenu selon la préparation XXXI dans 20 ml de diméthylformamide et on ajoute 0,156 g (5,2.10⁻³ mole) d'hydrure de sodium en suspension à 80 % dans l'huile. Après une heure sous agitation à température ambiante, on ajoute 2,05 g (4,8.10⁻³ mole) de l'ester méthylique de la N-[(3-bromométhyl-2,4-dichlorophényl)sulfonyl]-L-proline, en solution dans 25 ml de diméthylformamide. On agite le mélange réactionnel pendant 24 heures à température ambiante puis on ajoute doucement 250 ml d'eau. Le mélange est extrait par de l'éther éthylique plusieurs fois et les phases organiques rassemblées sont lavées à l'eau, séchées puis concentrées sous pression réduite. Le produit brut obtenu est purifié par chromatographie sur gel de silice avec pour éluant le mélange cyclohexane/acétate d'éthyle (70/30 ; v/v). On obtient ainsi 1,23 g du produit attendu sous forme d'un solide blanc (rendement = 45 %).
F = 164-165°C
[α]²¹_{D} = - 16° (c = 1,00 ; CH₂Cl₂)

### PREPARATION XXXIII

### N-[[3-[[2-méthyl-4-(trifluorométhyl)quinolin-8-yl]oxyméthyl]-2,4-dichlorophényl]sulfonyl]-L-proline

On prépare une solution de 1,08 g (1,9.10⁻³ mole) de l'ester obtenu selon la préparation XXXII dans 10 ml de méthanol et 10 ml d'une solution aqueuse d'hydroxyde de sodium 1N et on porte le mélange sous agitation à 90-100°C pendant 2 heures, puis on concentre le milieu réactionnel sous pression réduite. Le résidu est repris à l'eau, on ajoute de l'éther éthylique et on acidifie avec une solution diluée d'acide chlorhydrique jusqu'à pH 2,5. Après décantation et extraction de la phase aqueuse avec de l'éther éthylique, les phases organiques rassemblées sont lavées à l'eau, séchées puis concentrées sous pression réduite. Après purification par chromatographie sur gel de silice avec pour éluant un mélange d'acétate d'éthyle/méthanol (90/10 ; v/v), on obtient 0,95 g du produit attendu sous forme d'une poudre crème (rendement = 90 %)
F = 215°C
[α]²⁵_{D} = - 2,3° (c = 0,78 ; DMSO)

### PREPARATION XXXIV

### Acide [3-[(4-cyanobenzoyl)amino]propyl]carbamique, 1,1-diméthyléthyl ester

On prépare une solution de 10 g (57.10⁻³ mole) d'acide (3-aminopropyl)carbamique, 1,1-diméthyléthyl ester (ou N-Boc-1,3-propanediamine) dans 75 ml de dichlorométhane et on ajoute 15,9 ml (114.10⁻³ mole) de triéthylamine. On refroidit le mélange avec un bain de glace et ajoute progressivement 10,38 g (60.10⁻³ mole) du chlorure de l'acide 4-cyanobenzoïque. On laisse revenir le milieu réactionnel à température ambiante, le maintient sous agitation pendant 10 heures, puis le verse sur 150 ml d'eau, On extrait avec du dichlorométhane et la phase organique obtenue est lavée avec une solution d'acide chlorhydrique 1N, puis avec de l'eau et enfin séchée sur sulfate de- sodium et concentrée sous pression réduite. On obtient ainsi 16,4 g du produit attendu sous forme de cristaux ocre (rendement = 94 %).
RMN¹H (DMSO) : 1,37 (s,9H) ; 1,63 (m,2H) ; 2,97 (m,2H) ; 3,26 (m,2H) ; 6,84 (t,1H) ; 7,97 (s,4H) ; 8,70 (t,1H).

### PREPARATION XXXV

### Chlorhydrate de N-(3-aminopropyl)-4-cyanobenzamide

On dissout 16,4 g (54.10⁻³ mole) du composé obtenu selon la préparation III dans 150 ml d'acétate d'éthyle, puis on ajoute 104 ml d'une solution de chlorure d'hydrogène à 2,6 mole/l dans l'acétate d'éthyle. Le mélange est agité à température ambiante pendant 24 heures, puis concentré sous pression réduite. Le produit brut, ainsi obtenu, est lavé avec de l'éther éthylique et séché sous vide à 40°C. On obtient ainsi 12,15 g du produit attendu sous forme d'un solide blanc-cassé (rendement = 94 %).
F = 176-180°C

### PREPARATION XXXVI

### 1-[[3-[[2-méthyl-4-(trifluorométhyl)quinolin-8-yl]oxyméthyl]-2,4-dichlorophényl]sulfonyl]-N-[3-[(4-cyanophényl)carboxylamino]propyl]-2(S)-pyrrolidinecarboxamide

En opérant de façon analogue à la préparation V, au départ des composés obtenus selon la préparation XXXIII et XXXV et en présence de N-méthylmorpholine, on obtient le produit attendu sous forme de cristaux blancs (rendement = 53 %).
F = 92-94°C
[α]_{D}²⁴ = - 30° (c = 1,01 ; CHCl₃)

### PREPARATION XXXVII

### 1-[[3-[[2-méthyl-4-(trifluorométhyl)quinolin-8-yl]oxyméthyl]-2,4-dichlorophényl]sulfonyl]-N-[3-[[4-[(amino)(hydroxyimino)méthyl]phényl]carbonylamino]propyl]-2(S)-pyrrolidinecarboxamide

En opérant de façon analogue à la préparation II, au départ du composé obtenu selon la préparation XXXVI, on obtient le produit attendu sous forme de cristaux jaunes (rendement = 16 %).
F = 192-194°C
[α]_{D}²² = - 11° (c = 1,00 ; DMSO)

### PREPARATION XXXVIII

### 1-[[3-[[2-méthyl-4-(trifluorométhyl)quinolin-8-yl]oxyméthyl]-2,4-dichlorophényl]sulfonyl]-N-[3-[[4-[(amino)(acétoxyimino)méthyl]phényl]carbonylamino]propyl]-2(S)-pyrrolidinecarboxamide

En opérant de façon analogue à la préparation III, au départ des composés obtenus selon la préparation XXXVII, on obtient le produit attendu sous forme de cristaux blancs (rendement = 96 %).
F = 170-172°C
[α]_{D}²⁴ = - 13° (c = 0,49 ; DMSO)

### Exemple 32

### 1-[[3-[[2-méthyl-4-(trifluorométhyl)quinolin-8-yl]oxyméthyl]-2,4-dichlorophényl]sulfonyl]-N-[3-[[4-(aminoiminométhyl)phényl]carbonylamino]-propyl]-2(S)-pyrrolidinecarboxamide

En opérant de façon analogue à l'exemple 11, au départ des composés obtenus selon la préparation XXXVIII, on obtient le produit attendu sous forme d'un solide jaune pâle (rendement = 70 %).
F = 142-144°C
[α]_{D}²⁴ = - 35° (c = 0,95 ; CH₃OH)

### Exemple 33

### 1-[[3-[[2-méthyl-4-(trifluorométhyl)quinolin-8-yl]oxyméthyl]-2,4-dichlorophényl]sulfonyl]-N-[3-[[4-(aminoiminométhyl)phényl]carbonylamino]-propyl]-2(S)-pyrrolidinecarboxamide, méthanesulfonate

En opérant de façon analogue à l'exemple 27, au départ du composé obtenu selon l'exemple 32, on obtient après lyophilisation le produit attendu sous forme de flocons jaune pâle (rendement = 97 %).
F = 150-154°C
[α]_{D}²¹ = - 33,5 ° (c = 0,98 ; CH₃OH)

### PREPARATION XXXIX

### Acide 2,6-diméthyl-3-(chlorosulfonyl)benzoïque

On ajoute lentement 4 ml d'acide chlorosulfonique à 1 g (0,067 mole) d'acide 2,6-diméthylbenzoïque et on agite le mélange réactionnel pendant 1 heure à 55°C. Après refroidissement, le mélange est versé sur 100 ml d'eau glacée. Le précipité formé est filtré, lavé à l'eau froide puis séché dans un dessiccateur sous vide. On obtient ainsi 1,22 g de produit attendu sous forme d'un solide blanc (rendement = 73 %).
F = 146°C

### PREPARATION XL

### Acide 2,6-diméthyl-3-[[2(S)-(méthoxycarbonyl)pyrrolidin-1-yl]sulfonyl]benzoïque

En opérant de façon analogue à la préparation XIV, au départ du chlorure d'acide obtenu selon la préparation XXXIX et d'ester méthylique de la L-proline, on obtient le produit attendu sous forme d'un solide crème (rendement = 32 %).
F = 110°C
[α]_{D}²⁹ = - 30,5° (c = 0,60 ; CH₃OH)

### PREPARATION XLI

### N-[(2,4-diméthyl-3-(chlorocarbonyl)phényl)sulfonyl]-L-proline, méthyl ester

On prépare une suspension de 0,77 g (2,26.10⁻³ mole) d'acide obtenu selon la préparation XL dans 25 ml de toluène et on ajoute 0,30 g (2,5.10⁻³ mole) de chlorure de thionyle. Le mélange réactionnel est porté à reflux sous agitation pendant 4 heures puis concentré sous pression réduite. Le résidu huileux cristallise au refroidissement. On obtient ainsi 0,80 g du produit attendu sous forme d'un solide beige (rendement = 98 %).
F = 92°C
[α]_{D}²⁶ = - 13° (c = 0,33 ; CHCl₃)

### PREPARATION XLII

### N-[(2,4-diméthyl-3-(hydroxyméthyl)phényl)sulfonyl]-L-proline, méthyl ester

On prépare une solution de 0,83 g (2,31.10-3 mole) du chlorure d'acide obtenu selon la préparation XLI dans 30 ml de diglyme et on ajoute, à 80°C, 0,1 g de borohydrure de sodium par fractions. Le mélange réactionnel est agité pendant 30 minutes à 80°C puis refroidi. On ajoute de l'eau et on extrait 3 fois par de l'acétate d'éthyle. Les phases organiques rassemblées sont lavées à l'eau, séchées sur sulfate de magnésium puis concentrées sous pression réduite. Le produit brut est purifié par chromatographie sur gel de silice avec pour éluant le mélange dichlorométhane/acétate d'éthyle (9/1 ; v/v). On obtient ainsi 500 mg du produit attendu sous forme d'une huile (rendement = 69 %).
[α]_{D}²⁶ = - 28,5° (c = 0,66 ; CH₃OH)

### PREPARATION XLIII

### N-[[2,4-diméthyl-3-(méthylsulfonylméthyl)phényl]sulfonyl]-L-proline, méthyl ester

On refroidit à 0°C une solution de 0,46 g (1,4.10⁻³ mole) de l'alcool obtenu selon la préparation XLII dans 7 ml de dichlorométhane et on ajoute 0,356 g (3,5.10⁻³ mole) de triéthylamine puis 0,37 g (3,2.10⁻³ mole) de chlorure de méthanesulfonyle. On agite le mélange pendant 3 heures à température ambiante puis on le lave avec une solution de bicarbonate de sodium, puis avec de l'eau. La phase organique est séchée puis concentrée sous pression réduite. On obtient ainsi 0,57 g d'une huile qui contient majoritairement le produit attendu et le dérivé chlorométhylé correspondant. Cette huile est utilisée directement pour l'étape suivante.

### PREPARATION XLIV

### N-[[3-[(2,4-diméthylquinolin-8-yl)oxyméthyl]-2,4-diméthylphényl]sulfonyl]-L-proline, méthyl ester

On ajoute 45 mg (1,5.10⁻³ mole) d'hydrure de sodium (en suspension à 80 % dans l'huile) à une solution de 0,23 g (1,3.10⁻³ mole) de 2,4-diméthyl-8-hydroxyquinoléine dans 5 ml de diméthylformamide. Après agitation pendant 30 minutes à température ambiante, on ajoute 550 mg du composé obtenu selon la préparation XLIII en solution dans 3 ml de diméthylformamide. Après 3 heures sous agitation à température ambiante, le mélange réactionnel est versé sur 100 ml d'eau glacée et extrait 2 fois avec de l'acétate d'éthyle. Les phases organiques rassemblées sont lavées à l'eau, séchées sur sulfate de magnésium et concentrées sous pression réduite. Le résidu est purifié par chromatographie sur gel de silice avec pour éluant le mélange dichlorométhane/acétate d'éthyle (80/20 ; v/v). On obtient ainsi 367 mg du produit attendu sous forme d'un solide amorphe (rendement = 56 %).
F = 50°C
[α]_{D}²⁶ = - 15° (c = 0,39 ; CH₃OH)

### PREPARATION XLV

### N-[[3-[(2,4-diméthylquinolin-8-yl)oxyméthyl]-2,4-diméthylphényl]sulfonyl]-L-proline

En opérant de façon analogue à la préparation XXXIII, au départ du composé obtenu selon la préparation XLIV, on obtient le produit attendu sous forme d'un solide blanc (rendement = 88 %).
F = 136°C
[α]_{D}²⁶ = - 67° (c = 0,57 ; CH₃OH)

### PREPARATION XLVI

### 1-[[3-[(2,4-diméthylquinolin-8-yl)oxyméthyl]-2,4-diméthylphényl]sulfonyl]-N-[3-[(4-cyanophényl)carbonylamino]propyl]-2(S)-pyrrolidinecarboxamide

En opérant de façon analogue à la préparation V, au départ des composés obtenus selon les préparations XLV et XXXV, on obtient le produit attendu sous forme de cristaux blancs (rendement = 78 %).
F = 68°C
[α]_{D}²³ = - 28° (c = 0,33 ; CH₃OH)

### PREPARATION XLVII

### 1-[[3-[(2,4-diméthylquinolin-8-yl)oxyméthyl]-2,4-diméthylphényl]sulfonyl]-N-[3-[[4-[(amino)(hydroxyimino)méthyl]phényl]carbonylamino]propyl]-2(S)-pyrrolidinecarboxamide

En opérant de façon analogue à la préparation II, au départ du composé obtenu selon la préparation XLVI, on obtient le produit attendu sous forme d'un solide blanc (rendement = 93 %).
F = 130°C
[α]_{D}²³ = - 23° (c = 0,40 ; CH₃OH)

### PREPARATION XLVIII

### 1-[[3-[(2,4-diméthylquinolin-8-yl)oxyméthyl]-2,4-diméthylphényl]sulfonyl]-N-[3-[[4-[(amino)(acétoxyimino)méthyl]phényl]carbonylamino]propyl]-2(S)-pyrrolidinecarboxamide

En opérant de façon analogue à la préparation III, au départ du composé obtenu selon la préparation XLVII, on obtient le produit attendu sous forme de cristaux blancs (rendement = 93 %).
F = 60°C
[α]_{D}²³ = - 24° (c = 0,39 ; CH₃OH)

### Exemple 34

### 1-[[3-[(2,4-diméthylquinolin-8-yl)oxyméthyl]-2,4-diméthylphényl]sulfonyl]-N-[3-[[4-(aminoiminométhyl)phényl]carbonylamino]propyl]-2(S)-pyrrolidine-carboxamide

En opérant de façon analogue à l'exemple 11, au départ du composé obtenu selon la préparation XLVIII, on obtient le produit attendu sous forme d'un solide blanc (rendement = 79 %).
F = 150°C
[α]_{D}²³ = - 35° (c = 0,39 ; CH₃OH)

### Exemple 35

### 1-[[3-[(2,4-diméthylquinolin-8-yl)oxyméthyl]-2,4-diméthylphényl]sulfonyl]-N-[3-[[4-(aminoiminométhyl)phényl]carbonylamino]propyl]-2(S)-pyrrolidinecarboxamide, dichlorhydrate

On prépare une solution de 0,56 g (0,83.10⁻³ mole) du composé obtenu selon l'exemple 34, dans 4 ml de méthanol et on ajoute 1 ml d'une solution saturée de chlorure d'hydrogène dans l'éther éthylique. Après agitation du mélange pendant 15 mn, on ajoute 50 ml d'éther éthylique. Le précipité formé est filtré, lavé à l'éther, séché au dessiccateur puis dissous dans 10 ml d'eau. La solution obtenue est filtrée et lyophilisée. On obtient ainsi 0,58 g du produit attendu sous forme d'une poudre blanche (rendement = 94 %)
F = 195°C
[α]_{D}¹⁹ = - 86° (c = 0,47 ; CH₃OH)

### Exemple 36

### 1-[[3-[(2,4-diméthylquinolin-8-yl)oxyméthyl]-2,4-dichlorophényl]sulfonyl]-N-[3-[[4-(aminoiminométhyl)phényl]carbonylamino]propyl]-2(S)-pyrrolidinecarboxamide, di(méthanesulfonate)

On prépare une solution de 1,59 g (16,5.10⁻³ mole) d'acide méthanesulfonique dans 15 ml de méthanol et on ajoute doucement,sous agitation, une solutio dans le méthanol de 5,87 g (8,25.10⁻³ mole) du composé obtenu selon l'exemple 5. On maintient le mélange sous agitation pendant 30 minutes à température ambiante puis on verse la solution obtenue dans 600 ml d'éther éthylique. Le précipité blanc formé est filtré et lavé à l'éther sur le filtre, puis séché dans un dessiccateur sous vide et remis en solution dans 50 ml d'eau. La solution obtenue est filtrée puis lyophilisée. On obtient ainsi 6,6 g du produit attendu sous forme d'une poudre blanche (rendement = 88 %).
F = 174-176°C
[α]²⁴_{D} = - 29° (c = 0,30 ; CH₃OH)

L'activité des produits selon l'invention a été évaluée en fonction de leur aptitude à se lier aux récepteurs de la bradykinine. En effet, les kinines, dont le principal représentant est la bradykinine, forment un groupe de petits peptides qui contribuent de façon importante à la réponse inflammatoire et apparaissent de ce fait impliqués dans la pathophysiologie des maladies inflammatoires. De plus, la bradykinine est un des agents algésiants parmi les plus puissants connus. Les kinines activent deux types de récepteurs appelés respectivement B₁ et B₂. Le récepteur B₂ appartient à la grande famille des récepteurs à sept domaines transmembranaires couplés aux G-protéines. Nous décrivons dans la présente invention des composés se liant au récepteur B₂ et bloquant de ce fait la fixation de la bradykinine.

Le test pharmacologique utilisé est le suivant : des segments d'iléon de cobayes mâles [de souche Dunkin-Hartley (Iffa Credo, l'Arbresle, France)] sont isolés et broyés dans le tampon TES suivant : TES 25mM, 1,10-phénanthroline 1mM (pH 6.8), bacitracine 140 µg/ml, BSA 1g/l. Les membranes sont ensuite isolées par centrifugation (18000 tours par minute ; 20 min ; 4°C). Les études de liaison sont effectuées dans ce tampon TES en utilisant la [³H]-bradykinine (120 pM), 50 µg de protéine membranaire par essai (volume final 500 µl) avec un temps d'équilibre de 90 min à. 20°C. On détermine ensuite le taux (en pourcentage) d'inhibition de la fixation de [³H]-bradykinine en présence de l'un des composés selon l'invention à tester à une concentration de 10⁻⁶M.

Les résultats obtenus (notés "activité") lors de ces essais sont consignés dans le tableau I ci-après en regard des exemples figurant dans la description. Dans ce tableau, Pos indique la position du groupe amidine sur le cycle aromatique (ou hétéroaromatique) par rapport au groupe B, et dans la colonne "Sel" Chl signifie que le composé est sous forme de sel avec l'acide chlorhydrique et Ms sous forme de sel avec l'acide méthanesulfonique.

Les composés de la présente invention, qui inhibent la liaison de la [³H] bradykinine au récepteur B₂ de cobaye (voir tableau I), se lient également au récepteur B₂ humain cloné et transfecté de façon stable dans des cellules CHO (Chinese Hamster Ovary Cells). Ainsi dans ce test, certains composés inhibent à la concentration de 10 µM d'au moins 95 % la fixation de la [³H]-bradykinine au récepteur B₂.

Les composés de la présente invention peuvent être utiles dans le traitement des algies, et en particulier dans le traitement de nombreuses pathologies impliquant la bradykinine ou ses homologues. Parmi ces pathologies, on inclut les chocs septiques et hémorragiques, les réactions anaphylactiques, l'arthrose, la polyarthrite rhumatoïde, les rhinites, l'asthme, les maladies inflammatoires du tractus gastro-intestinal (par ex. colites, rectites, maladie de Crohn), la pancréatite, certains carcinomes, l'angiooedème héréditaire, la migraine, l'encéphalomyélite, la méningite, les accidents vasculaires cérébraux (notamment ceux provoqués par un choc traumatique cérébral), certains désordres neurologiques, les états inflammatoires vasculaires (par exemple : athérosclérose et artérite des membres inférieurs), les états douloureux (par exemple les céphalgies, les douleurs dentaires, les douleurs menstruelles), les contractions utérines prématurées, la cystite et les brûlures. Les composés selon l'invention peuvent également être utiles pour la potentialisation d'agents antiviraux.

Les composés de la présente invention, qui peuvent être utilisés sous forme de base libre ou de leurs sels d'addition non-toxiques, en association avec un excipient physiologiquement acceptable, sont en général prescrits en thérapeutique humaine à des doses d'environ 1 à 1000 mg/jour, sous une forme administrable par voie orale, par injection intraveineuse, intramusculaire ou sous-cutanée, par voie transdermique, par le moyen d'aérosols ou par le moyen de suppositoires.

Ces composés sont également administrables par voie topique, par exemple sous forme de gel ou de pommade.

Les composés de la présente invention trouvent également leur utilité, en tant que réactifs pharmacologiques, notamment pour l'étude des interactions hormone-récepteur. L'utilisation en tant que réactif pharmacologique peut faire appel à un dérivé radiomarqué de l'un des composés selon l'invention (par exemple avec du tritium [3H] ou du soufre [³⁵S]), dans le but d'obtenir un radio-ligand destiné à des études conformationnelles du récepteur B₂ de la bradykinine, ou des tests de fixation à ce type de récepteur, par exemple pour l'évaluation de nouveaux composés susceptibles de présenter une affinité pour le récepteur B₂ de la bradykinine.

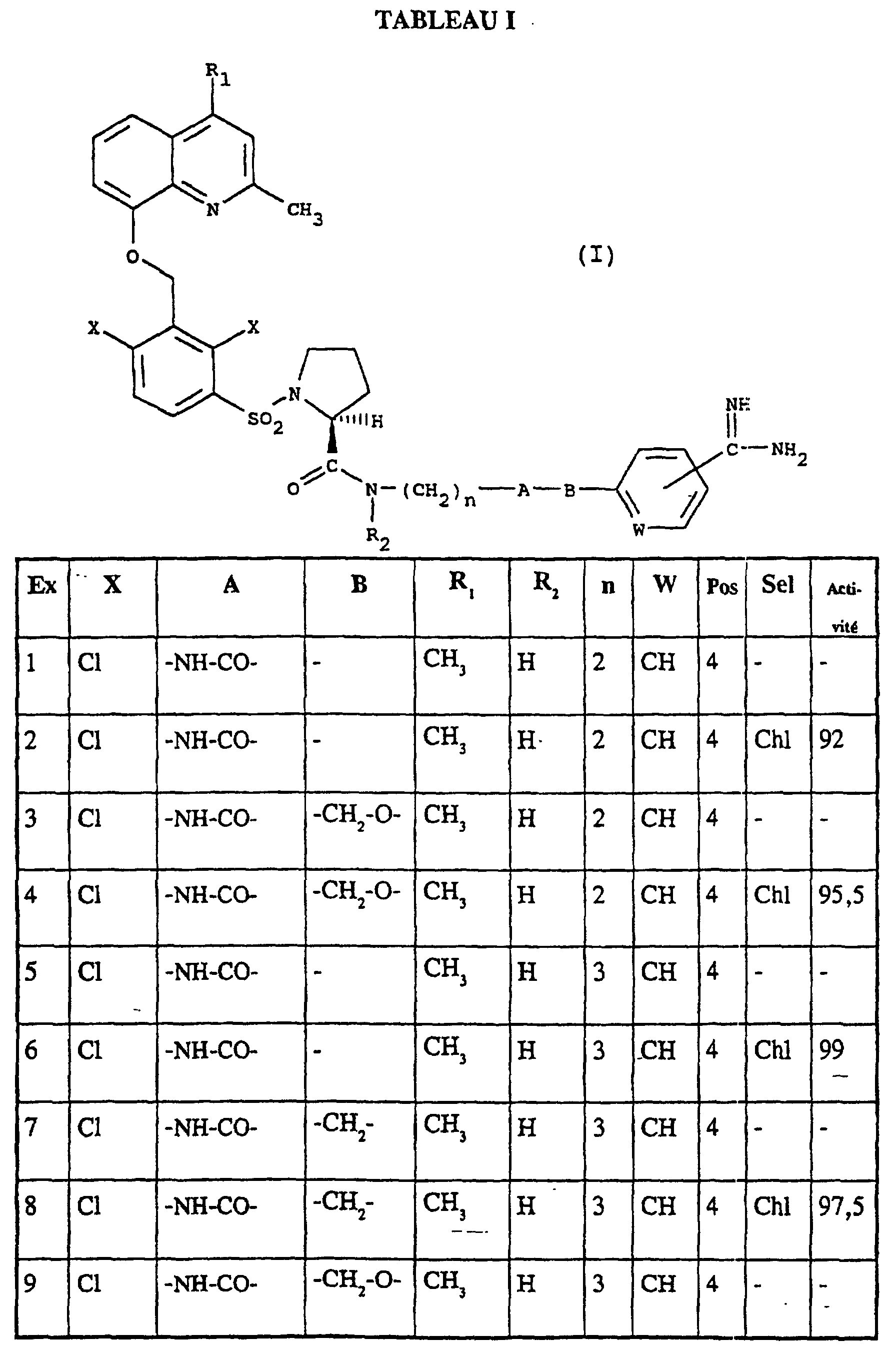

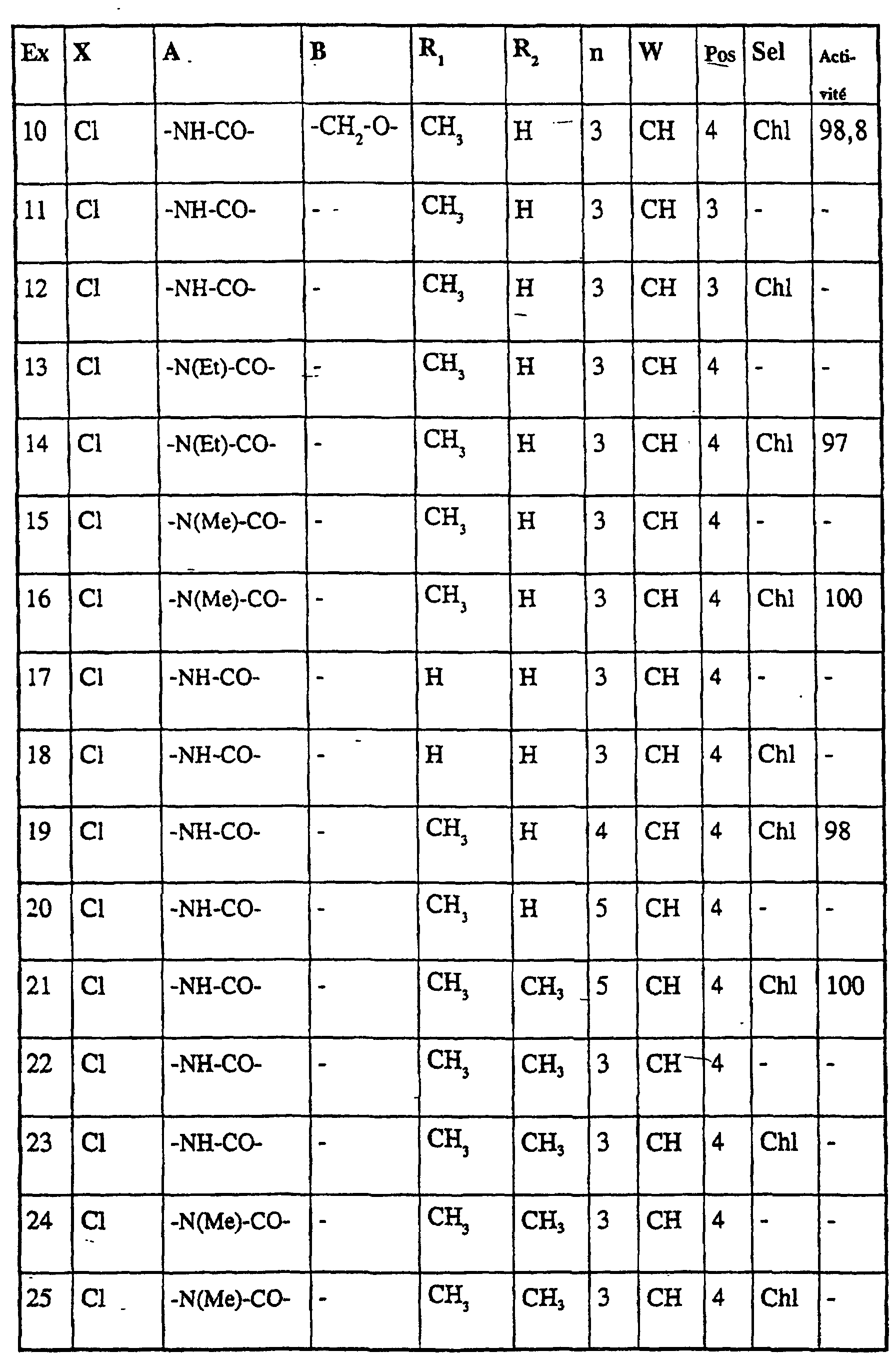

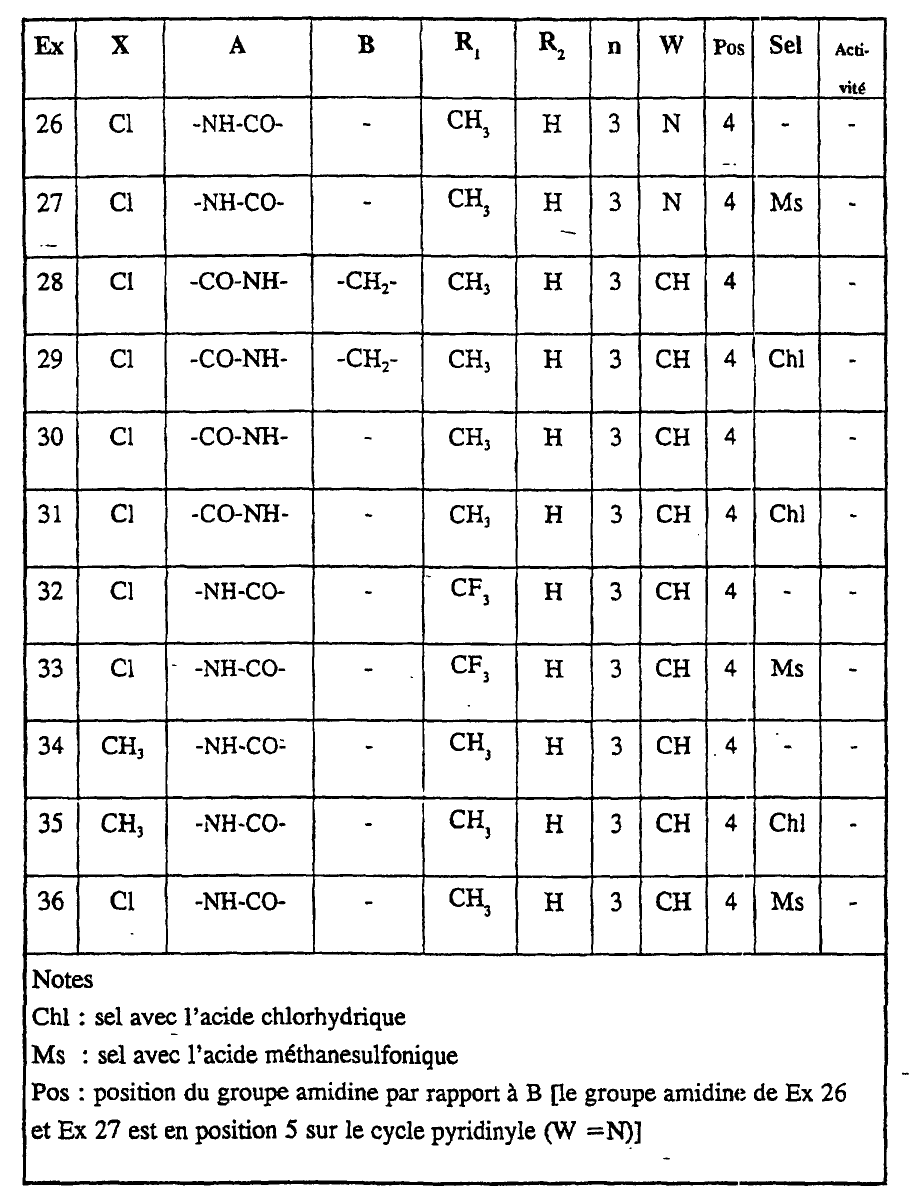

## Revendications

1. Composé de N-(benzènesulfonyl)-L-proline, **caractérisé en ce qu'**il est choisi parmi l'ensemble constitué par :
**(i)** les composés de formule I : 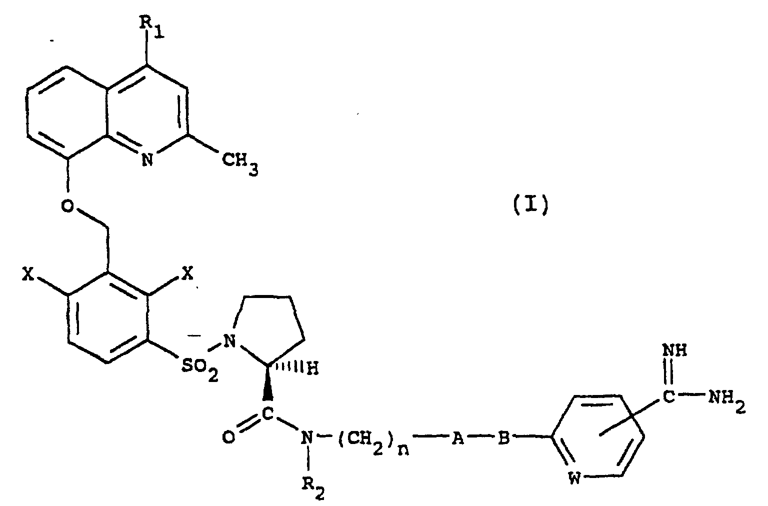 dans laquelle :
X représente un atome d'halogène ou un groupe méthyle,
A représente un groupe -N(R₃)-CO- ou -CO-N(R₃)-,
B représente une liaison simple, -CH₂- ou -CH₂-O-,
R₁ représente un atome d'hydrogène, un groupe alkyle en C₁-C₃ ou un groupe trifluorométhyle,
R₂ et R₃ représentent chacun indépendamment un atome d'hydrogène ou un groupe alkyle en C₁-C₃,
W représente CH ou N,
n représente 2, 3, 4 ou 5 ; et,
**(ii)** leurs sels d'addition.

2. Composé selon la revendication 1, **caractérisé en ce que** X est Cl.

3. Procédé de préparation d'un composé de formule I, **caractérisé en ce qu'**il comprend les étapes consistant à :
(1) faire réagir un acide de formule II : 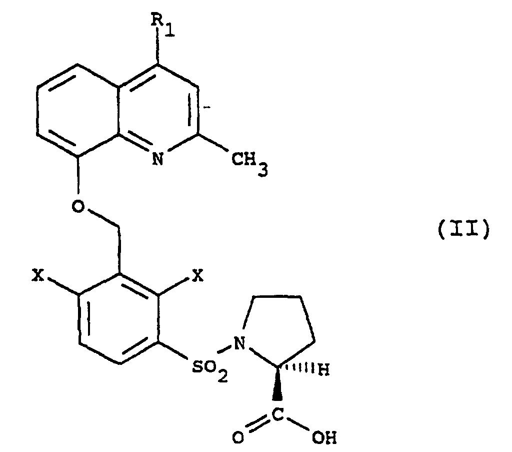 dans laquelle :
R₁ représente un atome d'hydrogène, un groupe alkyle en C₁-C₃ ou un groupe trifluorométhyle, et
X représente un halogène ou un groupe méthyle, avec une amine de formule III : 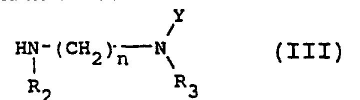
dans laquelle
R₂ et R₃ représentent chacun indépendamment H ou un groupe alkyle en C₁-C₃,
n représente 2, 3, 4 ou 5, et
Y représente un groupe amino-protecteur,
dans un solvant, en présence d'au moins un activateur couramment utilisé pour créer des liaisons de type peptidique, à une température proche de la température ambiante (comprise entre environ 0 et environ 40°C, et mieux, comprise entre 10 et 35°C), pendant 2 à 50 heures, pour obtenir un composé de formule IV: 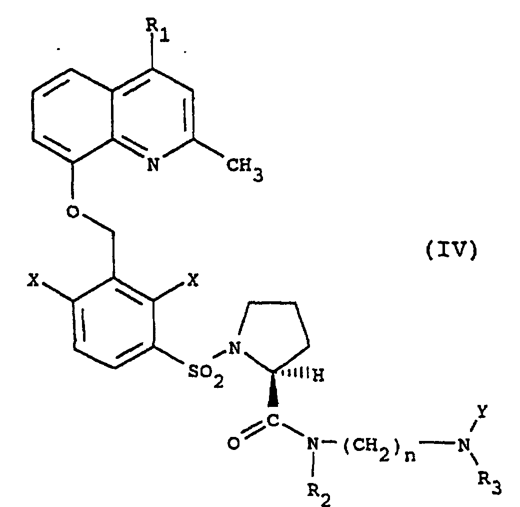 dans laquelle R₁, R₂ , R₃ , X, Y et n conservent la même signification que ci-dessus ;
(2) déprotéger le composé de formule IV, ainsi obtenu, de façon à remplacer le groupe protecteur Y par un atome d'hydrogène, dans un solvant, à température ambiante (15-25°C), pendant 5 à 30 heures, pour obtenir un composé de formule V : 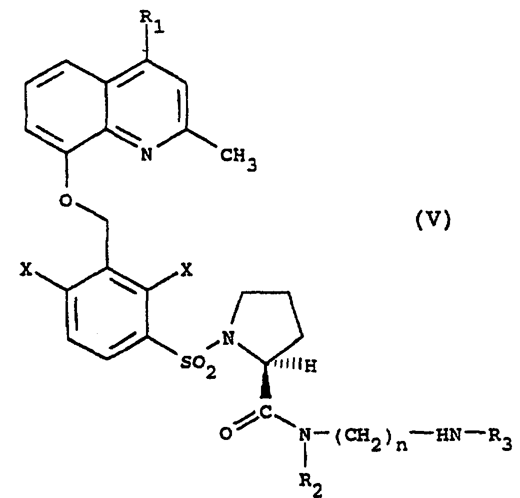 dans laquelle R₁, R₂ , R₃ , X et n conservent la même signification que ci-dessus ;
(3) faire réagir le composé de formule V, ainsi obtenu, avec un acide de formule VI: 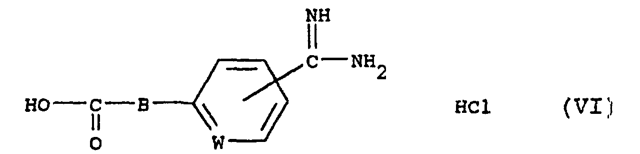 dans laquelle
B représente une liaison simple, un groupe -CH₂- ou -CH₂-O-, et
W représente -CH- ou un atome d'azote,
dans des conditions analogues à celles préconisées pour l'étape (1) ci-dessus, et, si nécessaire en présence d'une base si l'amine de formule V est mise en réaction sous sa forme salifiée, pour obtenir le composé de formule I : 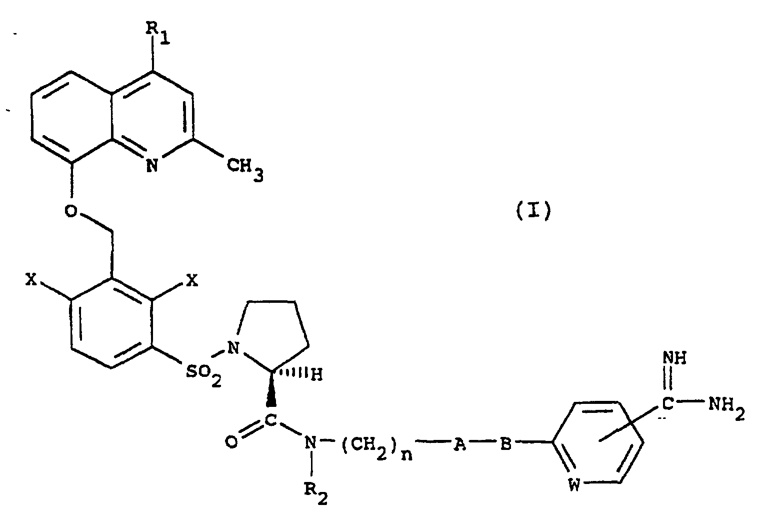 dans laquelle
A représente -N(R₃)-CO-,
B représente une liaison simple, -CH₂- ou -CH₂-O-,
R₁ représente H, un groupe alkyle en C₁-C₃ ou un groupe trifluorométhyle,
R₂ et R₃ représentent chacun indépendamment H ou un groupe alkyle en C₁-C₃,
X représente un atome d'halogène ou un groupe méthyle,
W représente CH ou N, et
n représente 2, 3, 4 ou 5 ; et,
(4) si nécessaire, faire réagir le composé de formule I, ainsi obtenu sous forme de base, avec un acide minéral ou organique pour obtenir ledit composé de formule I sous forme de sel d'addition.

4. Procédé de préparation d'un composé de formule I selon la revendication 3, **caractérisé en ce qu'**il comprend les étapes consistant à :
(a) faire réagir le composé de formule V, obtenu à l'étape (2) ci-dessus, avec un composé de formule VIII : 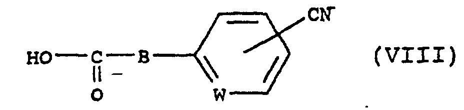 dans laquelle
B représente une liaison simple, -CH₂- ou -CH₂O-, et
W représente CH ou N,
dans des conditions opératoires analogues à celles de l'étape (3) décrite ci-dessus, pour obtenir un composé de formule IX : 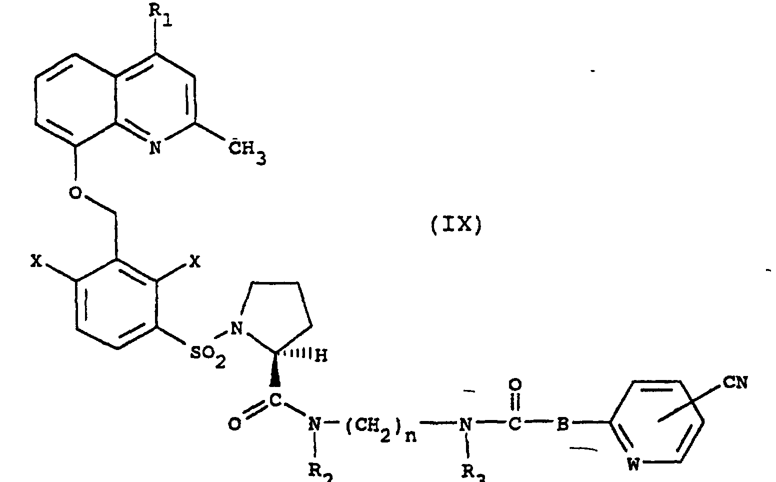 dans laquelle R₁, R₂ , R₃ , X, W, n et B conservent la même signification que dans les produits de départ ;
(b) faire réagir le composé de formule IX, ainsi obtenu, avec de l'hydroxylamine, dans un solvant, à température ambiante et pendant 2 à 12 heures, pour obtenir le composé de formule X : 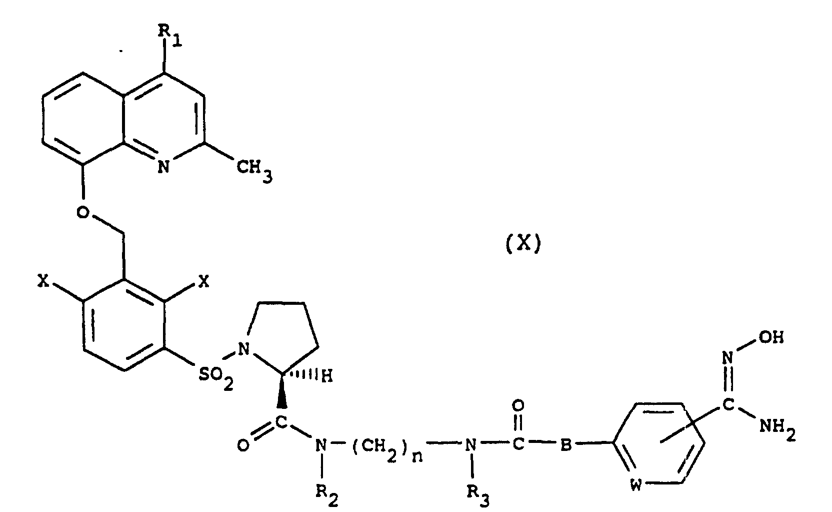 dans laquelle R₁, R₂ , R₃ , X, W, n et B conservent la même signification que précédemment ;
(c) faire réagir le composé de formule X, ainsi obtenu, avec un excès d'anhydride acétique, dans un solvant, à température ambiante pendant 1 à 15 heures, pour obtenir le composé de formule XI : 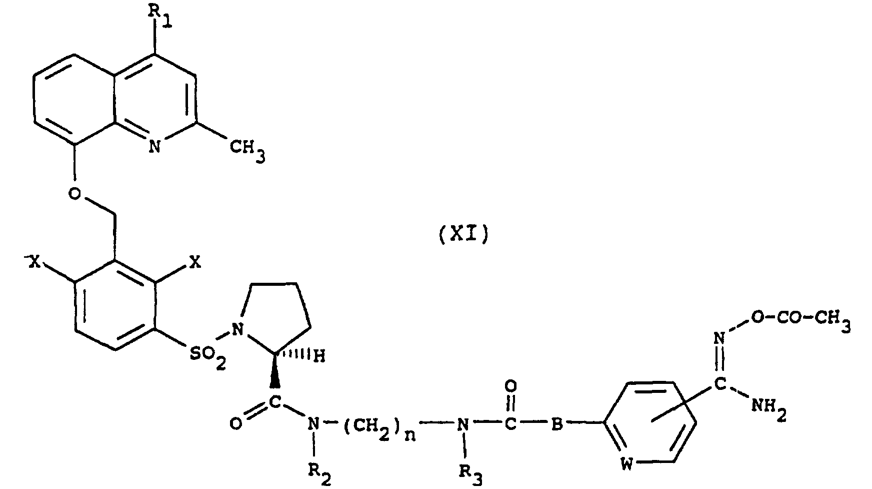 dans laquelle R₁, R₂, R₃, X, W, n et B conservent la même signification que ci-dessus ;
(d) hydrogéner catalytiquement le composé de formule XI, ainsi obtenu, dans un solvant, en présence d'un catalyseur d'hydrogénation, à température ambiante, sous une pression d'hydrogène comprise entre 10⁵ et 5.10⁵ Pa, pour obtenir le composé de formule I, dans laquelle R₁, R₂, R₃, X, W, n et B conservent la même signification que précédemment, A représente le groupe -N(R₃)-CO- et le groupe amidine conserve la position initiale du groupe cyano de l'acide de départ ; et,
(e) si nécessaire, faire réagir le composé de formule I, ainsi obtenu sous forme de base, avec un acide minéral ou organique pour obtenir ledit composé de formule I sous forme de sel d'addition.

5. Procédé selon la revendication 3, **caractérisé en ce que**, à l'étape (3),
(i) l'acide de formule VI est remplacé par le chlorure d'acide correspondant, et
(ii) la réaction est conduite dans un solvant en présence d'une base aprotique.

6. Composition thérapeutique **caractérisée en ce qu'**elle renferme, en association avec un excipient physiologiquement acceptable, au moins un composé choisi parmi l'ensemble constitué par les composés de formule I et leurs sels d'addition d'acide non-toxiques selon la revendication 1.

7. Utilisation d'une substance antagoniste d'un récepteur de la bradykinine et des hormones analogues, ladite utilisation étant **caractérisée en ce que** l'on fait appel à une substance antagoniste du récepteur B₂ de la bradykinine et choisie parmi l'ensemble constitué par les composés de formule I et leurs sels d'addition d'acide non-toxiques selon la revendication 1, pour l'obtention d'un médicament destiné à une utilisation en thérapeutique vis-à-vis d'états pathologiques impliquant la bradykinine ou ses homologues.

8. Utilisation selon la revendication 7, d'une substance choisie parmi l'ensemble constitué par les composés de formule I et leurs sels d'addition d'acide non-toxiques selon la revendication 1, pour l'obtention d'un médicament destiné à une utilisation en thérapeutique pour le traitement d'états douloureux.

9. Utilisation selon la revendication 7, d'une substance choisie parmi l'ensemble constitué par les composés de formule I et leurs sels. d'addition d'acide non-toxiques selon la revendication 1, pour l'obtention d'un médicament destiné à une utilisation en thérapeutique pour le traitement d'états inflammatoires.

10. Utilisation d'un composé de formule I ou l'un de ses sels d'addition d'acide selon la revendication 1 en tant que réactif pharmacologique.

## Patentansprüche

1. N-(Benzolsulfonyl)-L-prolin-Verbindung, **dadurch gekennzeichnet, dass** sie ausgewählt ist aus folgenden:
(i) Verbindungen der Formel 1: 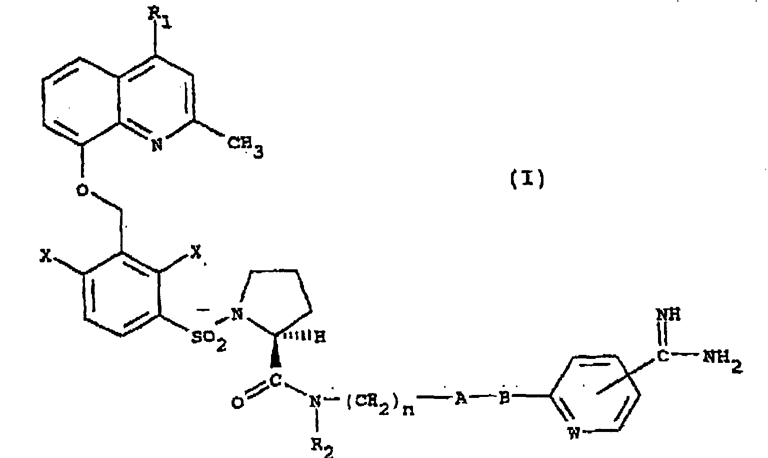 wobei
X ein Halogenatom oder eine Methylgruppe darstellt,
A einen Rest -N(R₃)-CO- oder -CO-N(R₃)- darstellt,
B eine Einfachbindung, -CH₂- oder -CH₂-O- darstellt,
R₁ ein Wasserstoffatom, einen C₁-C₃-Alkylrest oder eine Trifluormethylgruppe darstellt,
R₂ und R₃ jeweils unabhängig ein Wasserstoffatom oder einen C₁-C₃-Alkylrest darstellen,
W CH oder N darstellt,
n 2, 3, 4 oder 5 bedeutet; und
(ii) ihren Additionssalzen.

2. Verbindung nach Anspruch 1, **dadurch gekennzeichnet, dass** X für Cl steht.

3. Verfahren zur Herstellung einer Verbindung der Formel I, **dadurch gekennzeichnet, dass** es die folgenden Schritte umfasst:
(1) Umsetzen einer Säure der Formel II: 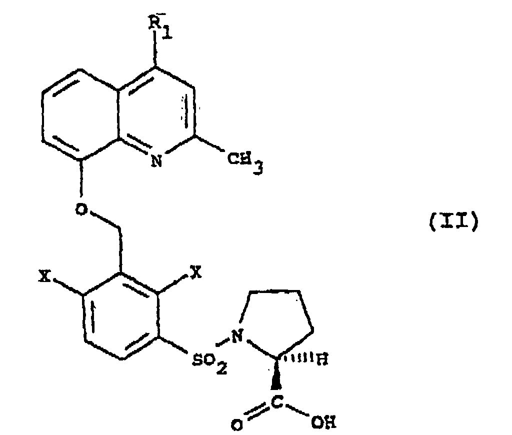 wobei:
R₁ ein Wasserstoffatom, einen C₁-C₃-Alkylrest oder eine Trifluormethylgruppe darstellt, und
X ein Halogenatom oder eine Methylgruppe darstellt,
mit einem Amin der Formel III: 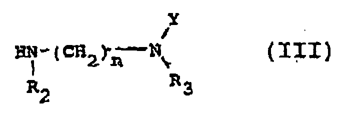
wobei
R₂ und R₃ jeweils unabhängig H oder einen C₁-C₃-Alkylrest darstellen,
n 2, 3, 4 oder 5 bedeutet, und
Y eine Aminoschutzgruppe darstellt,
in einem Lösungsmittel in Gegenwart mindestens eines gewöhnlich zum Erzeugen von Bindungen vom Peptid-Typ verwendeten Aktivators bei einer Temperatur nahe Umgebungstemperatur (zwischen etwa 0 und etwa 40°C und bevorzugt zwischen 10 und 35°C) über einen Zeitraum von 2 bis 50 Stunden, um eine Verbindung der Formel IV zu erhalten: 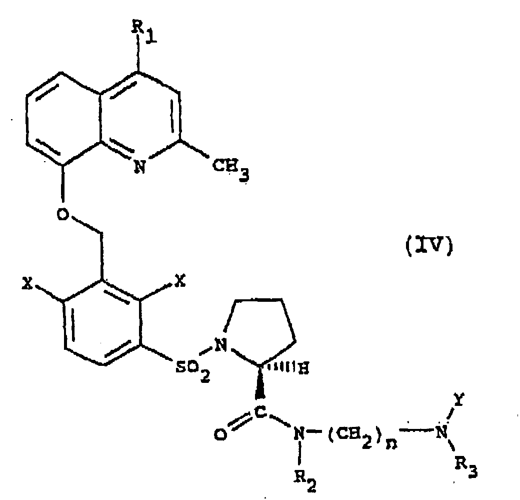 wobei R₁, R₂, R₃, X, Y und n die gleiche Bedeutung wie vorstehend beschrieben haben;
(2) Abspalten der Schutzgruppe von der so erhaltenen Verbindung der Formel IV, so dass die Schutzgruppe Y durch ein Wasserstoffatom ersetzt wird, in einem Lösungsmittel bei Umgebungstemperatur (15 bis 25°C) über einen Zeitraum von 5 bis 30 Stunden, um eine Verbindung der Formel V zu erhalten: 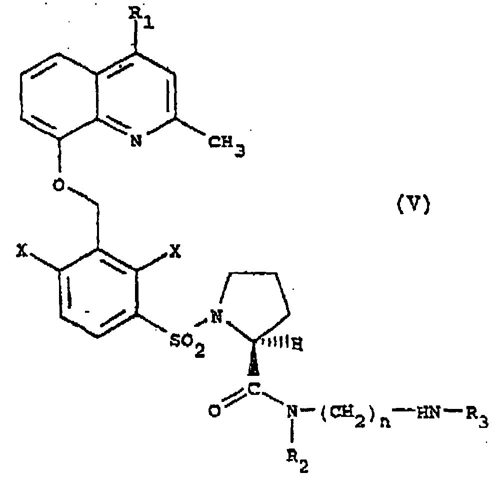 wobei R₁, R₂, R₃, X und n die gleiche Bedeutung wie vorstehend beschrieben haben;
(3) Umsetzen der so erhaltenen Verbindung der Formel V mit einer Säure der Formel VI: 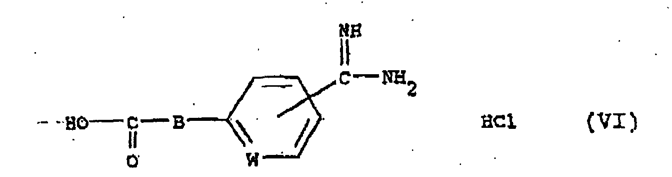 wobei
B eine Einfachbindung, einen Rest -CH₂- oder -CH₂-O- darstellt, und
W -CH- oder ein Stickstoffatom darstellt,
unter zu den vorstehend für Schritt (1) empfohlenen analogen Bedingungen und, falls erforderlich, in Gegenwart einer Base, wenn das Amin der Formel V in Form seines Salzes umgesetzt wird, um die Verbindung der Formel I zu erhalten: 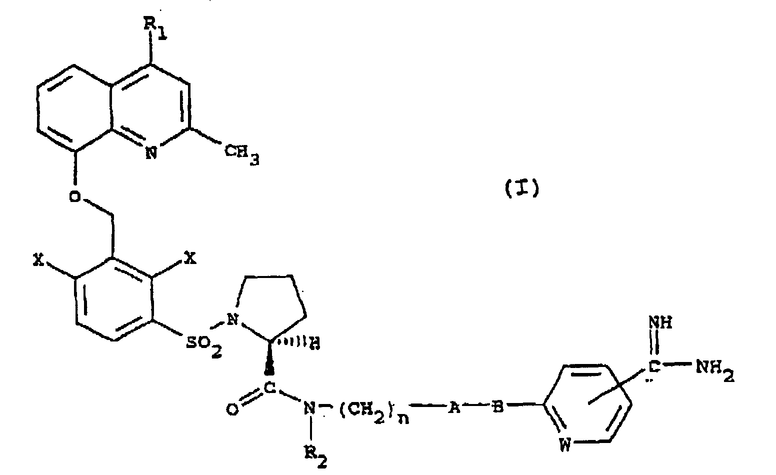 wobei
A -N(R₃)-CO- darstellt,
B eine Einfachbindung, -CH₂- oder -CH₂-O- darstellt,
R₁ H, einen C₁-C₃-Alkylrest oder eine Trifluormethylgruppe darstellt,
R₂ und R₃ jeweils unabhängig H oder einen C₁-C₃-Alkylrest darstellen,
X ein Halogenatom oder einen Methylrest darstellt,
W CH oder N darstellt, und
n 2, 3, 4 oder 5 bedeutet; und
(4) falls erforderlich Umsetzen der so in Form einer Base erhaltenen Verbindung der Formel I mit einer Mineralsäure oder organischen Säure, um die Verbindung der Formel I in Form des Additionssalzes zu erhalten.

4. Verfahren zur Herstellung einer Verbindung der Formel I nach Anspruch 3, **dadurch gekennzeichnet, dass** es die folgenden Schritte umfasst:
(a) Umsetzen der in vorstehendem Schritt (2) erhaltenen Verbindung der Formel V mit einer Verbindung der Formel VIII: 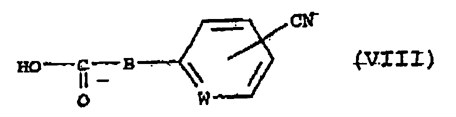 wobei
B eine Einfachbindung, -CH₂- oder -CH₂O- darstellt, und
W CH oder N darstellt,
unter zu den vorstehend für Schritt (3) beschriebenen analogen Betriebsbedingungen, um eine Verbindung der Formel IX zu erhalten: 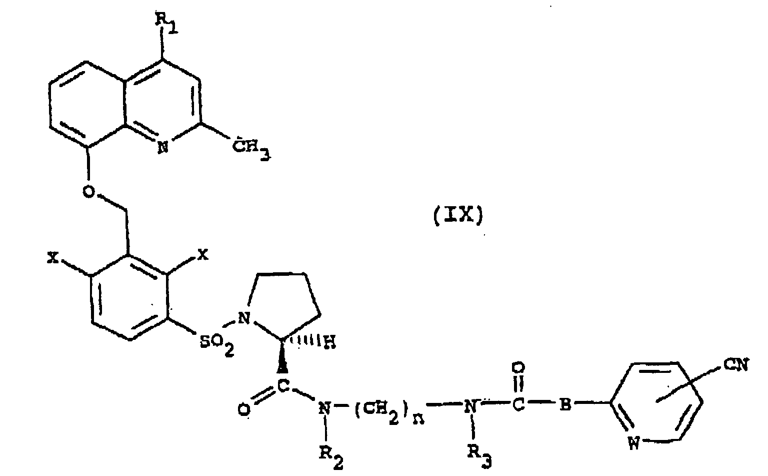 wobei R₁, R₂, R₃, X, W, n und B die gleiche Bedeutung wie in den Ausgangsprodukten haben;
(b) Umsetzen der so erhaltenen Verbindung der Formel IX mit Hydroxylamin in einem Lösungsmittel bei Umgebungstemperatur und über einen Zeitraum von 2 bis 12 Stunden, um die Verbindung der Formel X zu erhalten: 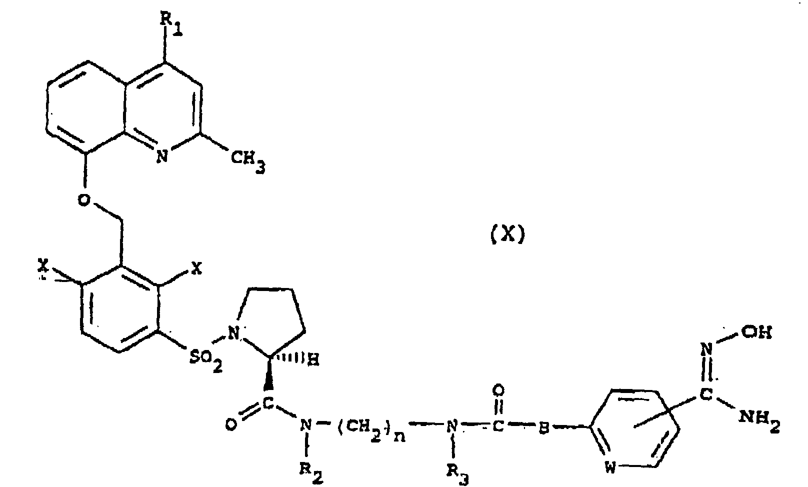 wobei R₁, R₂, R₃, X, W, n und B die gleiche Bedeutung wie vorstehend beschrieben haben;
(c) Umsetzen der so erhaltenen Verbindung der Formel X mit einem Übeischuss an Essigsäureanhydrid in einem Lösungsmittel bei Umgebungstemperatur über einen Zeitraum von 1 bis 15 Stunden, um die Verbindung der Formel XI zu erhalten: 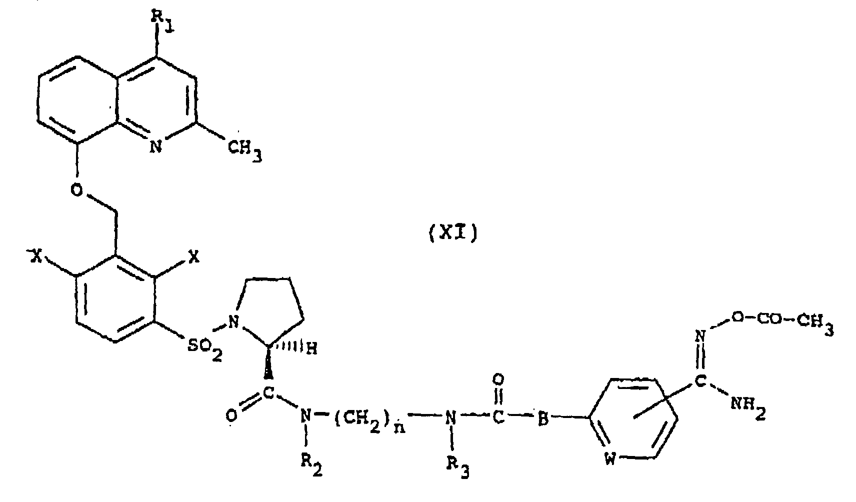 wobei R₁, R₂, R₃, X, W, n und B die gleiche Bedeutung wie vorstehend beschrieben haben;
(d) katalytisches Hydrieren der so erhaltenen Verbindung der Formel XI in einem Lösungsmittel in Gegenwart eines Hydrierungskatalysators bei Umgebungstemperatur unter einem Wasserstoftäruck zwischen 10⁵ und 5 x 10⁵ Pa, um die Verbindung der Formel I zu erhalten, wobei R₁, R₂, R₃, X, W, n und B die gleiche Bedeutung wie vorstehend beschrieben haben, A den Rest -N(R₃)-CO- darstellt und die Amidingruppe die anfängliche Position der Cyanogruppe der Säureabgangsgruppe beibehält; und
(e) falls erforderlich Umsetzen der so in Form einer Base erhaltenen Verbindung der Formel I mit einer Mineralsäure oder organischen Säure, um die Verbindung der Formel I in Form des Additionssalzes zu erhalten.

5. Verfahren nach Anspruch 3, **dadurch gekennzeichnet, dass** in Schritt (3)
(i) die Säure der Formel VI durch das entsprechende Säurechlorid ersetzt wird, und
(ii) die Umsetzung in einem Lösungsmittel in Gegenwart einer aprotischen Base durchgeführt wird.

6. Arzneimittel, **dadurch gekennzeichnet, dass** sie in Verbindung mit einem physiologisch verträglichen Exzipienten mindestens eine aus den Verbindungen der Formel I und deren nicht toxischen Säureadditionssalzen gemäß Anspruch 1 ausgewählte Verbindung beinhaltet.

7. Verwendung einer Substanz mit antagonistischer Wirkung gegen den Bradykinin-Rezeptor und analoge Hormone, wobei die Verwendung **dadurch gekennzeichnet ist, dass** man eine Substanz mit antagonistischer Wirkung gegen den Bradykinin-B₂-Rezeptor, die aus den Verbindungen der Formel I und deren nicht toxischen Säureadditionssalzen gemäß Anspruch 1 ausgewählt ist, verwendet, um ein Medikament zu erhalten, das zur therapeutischen Verwendung bei Krankheitszuständen, die mit Bradykinin oder dessen Homologen in Verbindung stehen, bestimmt ist.

8. Verwendung nach Anspruch 7 einer aus den Verbindungen der Formel I und deren nicht toxischen Säureadditionssalzen gemäß Anspruch 1 ausgewählten Substanz, um ein Medikament zu erhalten, das zur therapeutischen Verwendung zur Behandlung von Schmerzzuständen bestimmt ist.

9. Verwendung nach Anspruch 7 einer aus den Verbindungen der Formel I und deren nicht toxischen Säureadditionssalzen gemäß Anspruch 1 ausgewählten Substanz, um ein Medikament zu erhalten, das zur therapeutischen Verwendung zur Behandlung von Entzündungszuständen bestimmt ist.

10. Verwendung einer Verbindung der Formel I oder eines ihrer Säureadditionssalze gemäß Anspruch 1 als pharmakologischen Wirkstoff.

## Claims

1. An N-(benzenesulfonyl)-L-proline compound selected from the group consisting of:
**(i)** compounds of formula I: 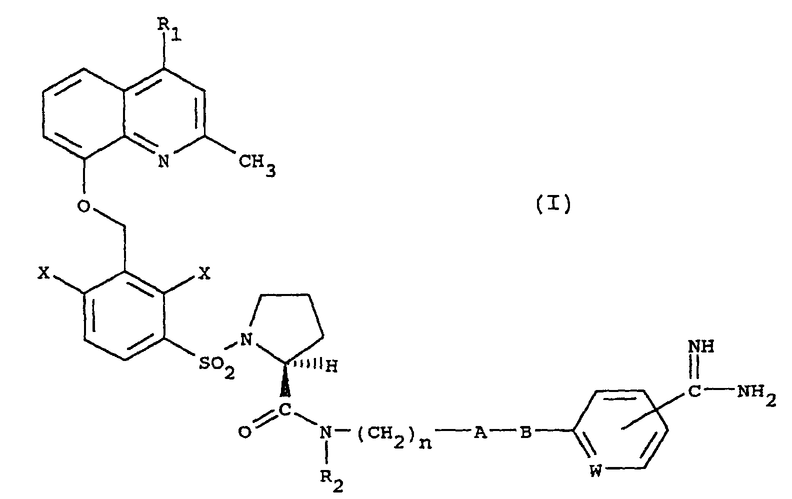 in which:
X is a halogen atom or a methyl group,
A is a -N(R₃)-CO- or -CO-N(R₃)- group,
B is a single bond, -CH₂- or -CH₂-O-,
R₁ is a hydrogen atom, a C₁-C₃ alkyl group or a trifluoromethyl group,
R₂ and R₃ are each independently a hydrogen atom or a C₁-C₃ alkyl group,
W is CH or N, and
n is 2, 3, 4 or 5; and
**(ii)** their addition salts.

2. A compound according to claim 1 wherein X is Cl.

3. A process for the preparation of a compound of formula I, said process comprising the steps which consist in:
(1) reacting an acid of formula II: 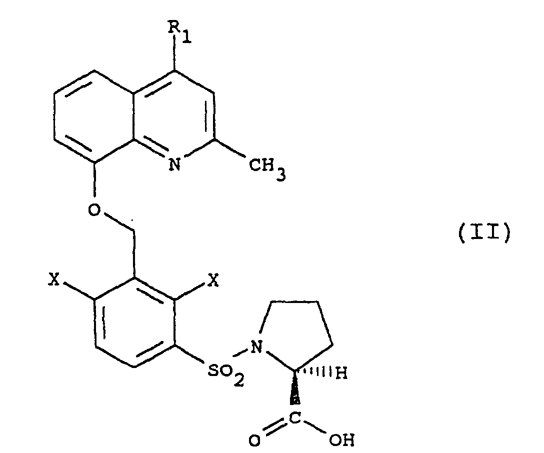 in which:
R₁ is a hydrogen atom, a C₁-C₃ alkyl group or a trifluoromethyl group, and
X is a halogen or a methyl group,
with an amine of formula III: 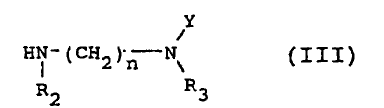 in which:
R₂ and R₃ are each independently H or a C₁-C₃ alkyl group,
n is 2, 3, 4 or 5, and
Y is an amino-protecting group,
in a solvent, in the presence of at least one activator commonly used to create linkages of the peptide type, at a temperature near room temperature (between about 0 and about 40°C and preferably between 10 and 35°C), for 2 to 50 hours, to give a compound of formula IV: 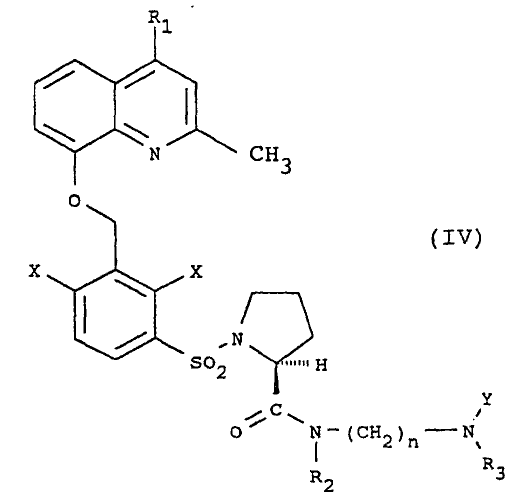 in which R₁, R₂, R₃, X, Y and n are as defined above;
(2) deprotecting the resulting compound of formula IV so as to replace the protecting group Y with a hydrogen atom, in a solvent, at room temperature (15-25°C), for 5 to 30 hours, to give a compound of formula V: 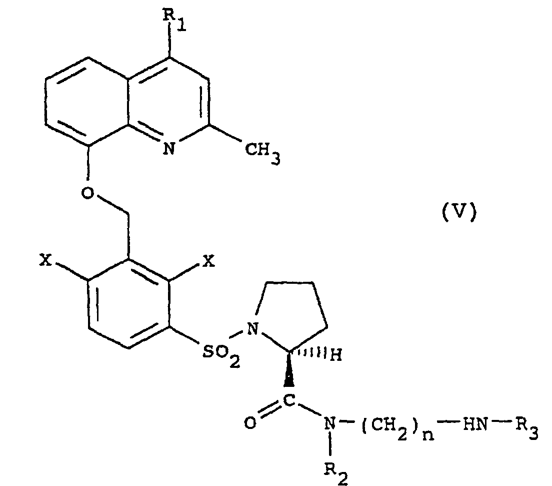 in which R₁, R₂, R₃, X and n are as defined above;
(3) reacting the resulting compound of formula V with an acid of formula VI: 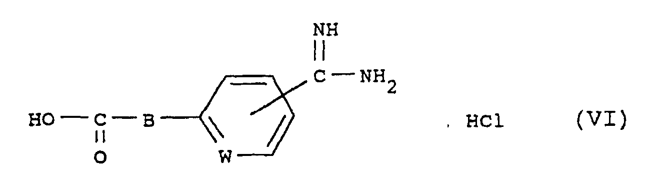 in which:
B is a single bond or a group -CH₂- or -CH₂-O-, and
W is -CH- or a nitrogen atom,
under conditions analogous to those recommended for step (1) above, and, if necessary, in the presence of a base if the amine of formula V is reacted in its salified form, to give the compound of formula I: 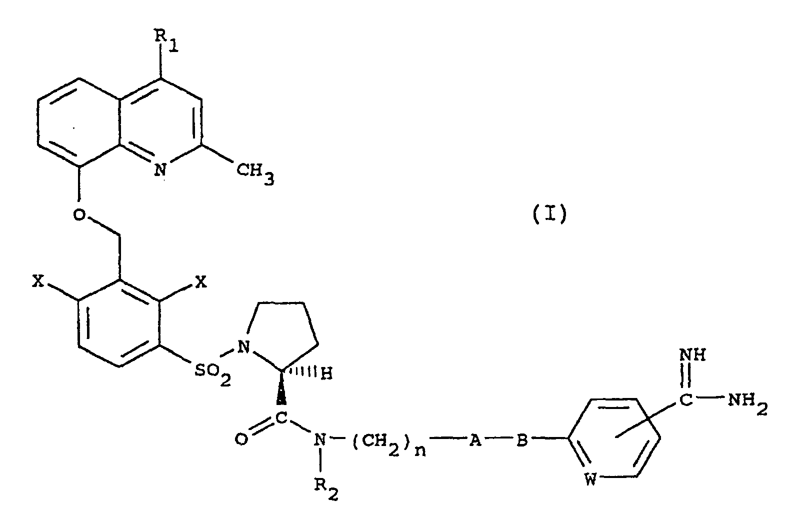 in which:
A is -N(R₃)-CO-,
B is a single bond, -CH₂- or -CH₂-O-,
R₁ is H, a C₁-C₃ alkyl group or a trifluoromethyl group,
R₂ and R₃ are each independently H or a C₁-C₃ alkyl group,
X is a halogen atom or a methyl group,
W is CH or N, and
n is 2, 3, 4 or 5; and
(4) if necessary, reacting the resulting compound of formula I, in the form of the base, with a mineral or organic acid to give said compound of formula I in the form of an addition salt.

4. A process for the preparation of a compound of formula I according to claim 3, said process comprising the steps which consist in:
(a) reacting the compound of formula V, obtained in step (2) above, with a compound of formula VIII: 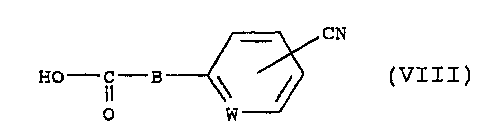 in which:
B is a single bond, -CH₂- or -CH₂O-, and
W is CH or N,
under operating conditions analogous to those of step (3) described above, to give a compound of formula IX: 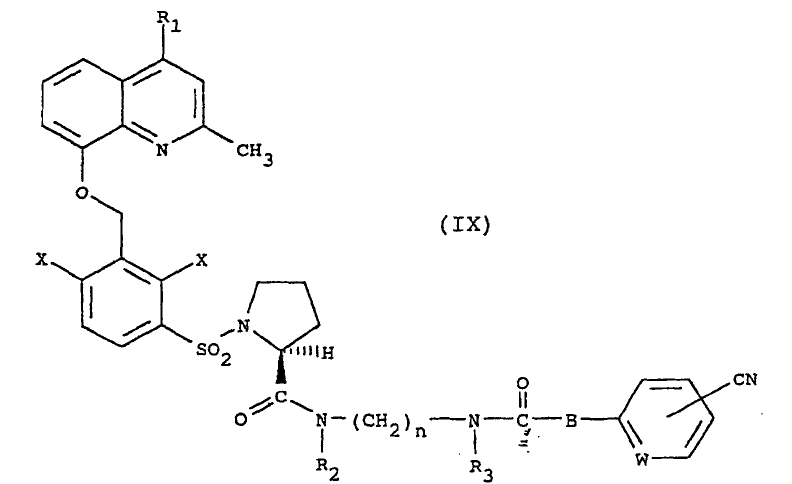 in which R₁, R₂, R₃, X, W, n and B are as defined in the starting materials;
(b) reacting the resulting compound of formula IX with hydroxylamine, in a solvent, at room temperature, for 2 to 12 hours, to give a compound of formula X: 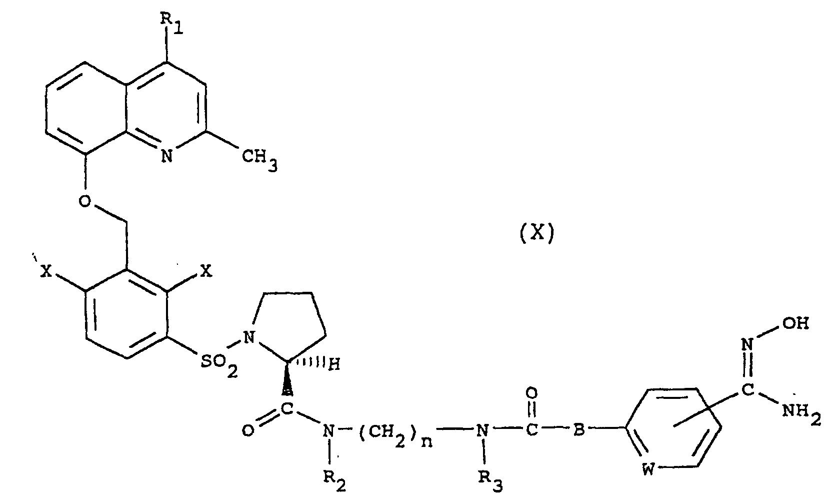 in which R₁, R₂, R₃, X, W, n and B are as defined above;
(c) reacting the resulting compound of formula X with excess acetic anhydride in a solvent, at room temperature, for 1 to 15 hours, to give a compound of formula XI: 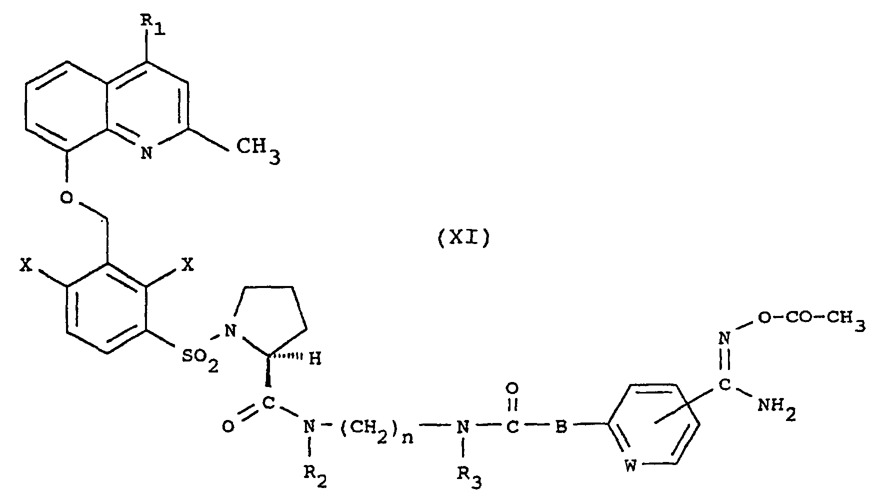 in which R₁, R₂, R₃, X, W, n and B are as defined above;
(d) catalytically hydrogenating the resulting compound of formula XI in a solvent, in the presence of a hydrogenation catalyst, at room temperature, under a hydrogen pressure of between 10⁵ and 5x10⁵ Pa, to give the compound of formula I in which R₁, R₂, R₃, X, W, n and B are as defined above, A is the group -N(R₃)-CO- and the amidine group retains the initial position of the cyano group of the starting acid; and
(e) if necessary, reacting the resulting compound of formula I, in the form of the base, with a mineral or organic acid to give said compound of formula I in the form of an addition salt.

5. A process according to claim 3 wherein, in step (3), (i) the acid of formula VI is replaced with the corresponding acid chloride, and (ii) the reaction is carried out in a solvent in the presence of an aprotic base.

6. A therapeutic composition containing, in association with a physiologically acceptable excipient, at least one compound selected from the group consisting of the compounds of formula I and their non-toxic acid addition salts according to claim 1.

7. Use of an antagonist of a receptor of bradykinin and analogous hormones, wherein a bradykinin B₂ receptor antagonist selected from the group consisting of the compounds of formula I and their non-toxic acid addition salts according to claim 1 is used to obtain a drug intended for use in therapeutics to combat pathological conditions involving bradykinin or its homologs.

8. Use according to claim 7 of a substance selected from the group consisting of the compounds of formula I and their non-toxic acid addition salts according to claim 1 for obtaining a drug intended for use in therapeutics for the treatment of painful states.

9. Use according to claim 7 of a substance selected from the group consisting of the compounds of formula I and their non-toxic acid addition salts according to claim 1 for obtaining a drug intended for use in therapeutics for the treatment of inflammatory states.

10. Use of a compound of formula I or one of its acid addition salts according to claim 1 as a pharmacological reagent.
